(19) Europäisches Patentamt / European Patent Office / Office européen des brevets

(11) **EP 4 157 065 B1**

(12) **EUROPEAN PATENT SPECIFICATION**

(45) Date of publication and mention
of the grant of the patent:
**17.09.2025   Bulletin 2025/38**

(21) Application number: **21710513.9**

(22) Date of filing: **12.03.2021**

(51) International Patent Classification (IPC):
***A61B 3/113*** *(2006.01)*

(52) Cooperative Patent Classification (CPC):
**A61B 3/113; G02B 27/0093; G02B 27/017;
G06F 3/013; G06V 40/19; G06V 40/193;**
G02B 2027/0138

(86) International application number:
**PCT/EP2021/056348**

(87) International publication number:
**WO 2021/239284 (02.12.2021 Gazette 2021/48)**

(54) **METHODS, DEVICES AND SYSTEMS ENABLING DETERMINATION OF EYE STATE VARIABLES**

VERFAHREN, VORRICHTUNGEN UND SYSTEME ZUR BESTIMMUNG VON
AUGENZUSTANDSVARIABLEN

PROCÉDÉS, DISPOSITIFS ET SYSTÈMES PERMETTANT LA DÉTERMINATION DE VARIABLES
D'ÉTAT DE L'OEIL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **26.05.2020   PCT/EP2020/064593**

(43) Date of publication of application:
**05.04.2023   Bulletin 2023/14**

(73) Proprietor: **Pupil Labs GmbH
12047 Berlin (DE)**

(72) Inventors:
• **PETERSCH, Bernhard
  12051 Berlin (DE)**
• **DIERKES, Kai
  10245 Berlin (DE)**

(74) Representative: **Zimmermann & Partner
Patentanwälte mbB
Postfach 330 920
80069 München (DE)**

(56) References cited:
**WO-A1-2020/244752**

• **SWIRSKI L ET AL: "A fully-automatic, temporal
approach to single camera, glint-free 3D eye
model fitting", INTERNET CITATION, 1 January
2013 (2013-01-01), pages 1 - 10, XP002785718,
Retrieved from the Internet <URL:https://pdfs.
semanticscholar.org/f0c0/
bc74c1dc11e8a09fa4bd4c300cf21d7cc96d.pdf>
[retrieved on 20181016]**
• **KRZYSZTOF KREJTZ ET AL: "A fast approach to
refraction-aware eye-model fitting and gaze
prediction", PROCEEDINGS OF THE 11TH ACM
SYMPOSIUM ON EYE TRACKING RESEARCH &
APPLICATIONS , ETRA '19, 25 June 2019
(2019-06-25), New York, New York, USA, pages 1 -
9, XP055724189, ISBN: 978-1-4503-6709-7, DOI:
10.1145/3314111.3319819**

**Description**

**TECHNICAL FIELD**

Embodiments of the present invention relate to methods, devices and systems that may be used in the context of eye tracking, in particular methods for generating data suitable for and enabling determining a state of an eye of a human or animal subject.

**BACKGROUND**

Over the last decades, camera-based eye trackers have become a potent and wide-spread research tool in many fields including human-computer interaction, psychology, and market research. Offering increased mobility compared to remote eye-tracking solutions, head-mounted eye trackers, in particular, have enabled the acquisition of gaze data during dynamic activities also in outdoor environments. The traditional computational pipeline for mobile gaze estimation using head-worn eye trackers involves eye landmark detection, in particular detecting the pupil center or ellipse fitting either using special-purpose image processing techniques or machine learning, and gaze mapping, traditionally using a geometric eye model or by directly mapping 2D pupil positions to 3D gaze directions or points, or 2D gaze points within a camera image of a likewise head-worn front facing scene camera. While the latter approach works well when calibrating the eye tracking device to a particular user and wearing state, once the head-worn eye tracker slightly slips or moves with respect to its initial, calibrated position on the head of the user, the calibrated mapping from 2D pupil positions to gaze points deteriorates or breaks down entirely. In order to cope with such eye tracker "slippage", eye tracking strategies using full 3D eye models are superior, since they allow to constantly recalculate the actual 3D location of the eye(s) (eyeball centers) with respect to the head-worn eye tracker, in particular with respect to the coordinate system(s) defined by the camera(s) recording the eye(s), and corresponding gaze vectors. Knowing the location/coordinates of the eyeball center also opens the way to pupillometry, i.e. measuring the actual size of the pupil.

Methods employing 3D eye models can in turn be divided into methods making use of corneal reflections - so called "glints" - produced by light sources located at known positions with respect to the cameras recording the eye images, and methods which instead derive the eye model location and gaze direction directly from the pupil shape, without the use of any artificially produced reflections.

Eye trackers using glints rely on complex optical setups involving the active generation of said corneal reflections by means of infrared (IR) LEDs and/or pairs of calibrated stereo cameras. Glint-based (i.e. using corneal reflections) gaze estimation needs to reliably detect those reflections in the camera image and needs to be able to associate each with a unique light source. If successful, the 3D position of the cornea center (assuming a known radius of curvature, i.e. a parameter of a 3D eye model) can be determined. Beside the additional hardware requirements, another issue encountered in this approach are spurious reflections produced by other illuminators, which may strongly impact the achievable accuracy. From an engineering point of view, glint-free estimation of gaze-related and other eye state variables of an eye is therefore highly desirable. However, determining of eye state variables from camera images alone (solving an inverse problem) is challenging and so far requires comparatively high computing power often limiting the application area, in particular if head and/or eye movement with respect to the camera is to be compensated (e.g. "slippage" of a head-mounted eye tracker). Head-mounted eye trackers are in general desired to resolve ambiguities during eye state estimation with more restricted hardware setups than remote eye-trackers.

In an alternative to "glint-based" methods for eye state estimation, methods which instead derive a 3D eye model location and gaze direction directly from the pupil shape, without the use of any artificially produced reflections exist, see for example reference [1]. One of the challenges of such methods is the size-distance ambiguity: given only one 2D image of an eye it is not possible to know a priori whether the pupil of the eye is small and close or large and far away. Resolving this ambiguity requires a time series of many camera images which show the eye under largely varying gaze angles with respect to the camera, and complex numerical optimization methods to fit the 3D eye model in an iterative fashion to said time series of eye observations to yield the final eyeball center coordinates in camera coordinate space, which in turn are needed to derive quantities like the 3D gaze vector or the pupil size in physical units, such as millimeters.

A simpler and faster ways of calculating the eyeball center and thus resolving the size-distance ambiguity without requiring computationally expensive iterative numerical optimization methods have been proposed in [4], see also WO2020/244752, and WO2020/244971. The methods described therein employ the same 3D eye model as [1], which does not include a cornea and has a single parameter, namely the distance R between eyeball center and pupil center, which can be assumed as a physiological constant since human eyes vary only to a small extent between individuals. A post-hoc refraction correction strategy to deal with the effects of corneal refraction is described in [4] and WO2020/244752. While this method of dealing with corneal refraction has been shown to work well, it requires ray-tracing of a substantial amount of synthetic images. Also, generation of the required polynomial features from the preliminary values of the eye state at a given point in time (one eye observation) during runtime and application of the correction mapping does take some calculation time. While the method is able to perform at common frame rates used in real-time applications, saving computational time and energy in mobile, real-time applications is always a prime directive and even faster methods are thus desirable.

Pupillometry - the study of temporal changes in pupil diameter as a function of external light stimuli or cognitive processing - is another field of application of general purpose eye-trackers and requires accurate measurements of pupil dilation. Average human pupil diameters are of the order of 3mm (size of the aperture stop), while peak dilation in cognitive processes can amount to merely a few percent with respect to a baseline pupil size, thus demanding for sub-millimeter accuracy. Video-based eye trackers are in general able to provide apparent (entrance) pupil size signals. However, the latter are usually subject to pupil foreshortening errors - the combined effect of the change of apparent pupil size as the eye rotates away from or towards the camera and the gaze-angle dependent influence of corneal refraction. Such errors can easily amount to more than 10%, thus being larger than the pupil size changes that need to be measured. Also, many prior art methods and devices only provide pupil size in (pixel-based) arbitrary units, while there is an inherent merit in providing an absolute value in units of physical length (e.g. [mm]), since cognitively induced absolute changes are largely independent of baseline pupil radius, and hence only measuring absolute values makes experiments comparable. Hence, only a 3D eye model based eye state determination which takes effects of corneal refraction into account is maximally useful for the purpose of precision pupillometry.

Accordingly, there is a need to further improve the speed, robustness and accuracy of the detection of eyeball position, gaze direction, pupil size and other eye state variables and reduce the computational effort required therefor, while taking into account the effects of corneal refraction.

Kai Dierkes et al.: "A fast approach to refraction-aware eye-model fitting and gaze prediction", ETRA '19: Proceedings of the 11th ACM Symposium on Eye Tracking Research & Applications Article No.: 23, pages 1 - 9 (DOI: 10.1145/3314111.3319819), discloses a method for eye tracking comprising: taking into account corneal refraction; using synthetic images generated with a two-sphere eye model; determining correction functions to account for refraction effects; fast-fitting non-iterative approach to eye model.

## SUMMARY

According to an embodiment of a method for generating data suitable for determining at least one eye state variable of at least one eye of a subject, the eye comprising an eyeball, an iris defining a pupil, and a cornea, the at least one eye state variable being derivable from at least one image of the eye taken with a camera of known camera intrinsics, the method includes providing a first 3D eye model modeling corneal refraction. Using the known camera intrinsics, synthetic image data of several model eyes according to the first 3D eye model is generated for a plurality of given values of at least one eye state variable. Using a given algorithm the at least one eye state variable is calculated using one or more of the synthetic images and a further 3D eye model having at least one parameter. A characteristic of a pupil image (image of the pupil) within each of the synthetic images is determined and one or more hypothetically optimal values of the at least one parameter of the further 3D eye model that minimize the error between the value(s) of the at least one given eye state variable and the value(s) of the corresponding eye state variable obtained when applying the given algorithm are determined. Finally, a relationship between the one or more hypothetically optimal values of the at least one parameter of the further 3D eye model and the characteristic of the pupil image is established.

According to an embodiment of a method for determining at least one eye state variable of at least one eye of a subject, the eye comprising an eyeball, an iris defining a pupil, and a cornea, the at least one eye state variable being derivable from at least one image of the eye taken with a camera of known camera intrinsics, the method comprises receiving image data of the at least one eye from a camera of known camera intrinsics and defining an image plane, determining a characteristic of a pupil image (image of the pupil) within the image data, providing a 3D eye model having at least one parameter, the parameter depending in a pre-determined relationship on the characteristic and using a given algorithm to calculate the at least one eye state variable using the image data and the 3D eye model including the at least one characteristic-dependent parameter.

According to an embodiment of a system for generating data suitable for determining at least one eye state variable of at least one eye of a subject, the eye comprising an eyeball, an iris defining a pupil, and a cornea, the at least one eye state variable being derivable from at least one image of the eye taken with a camera of known camera intrinsics, the system comprises a computing and control unit configured to generate, using the known camera intrinsics, synthetic image data of several model eyes according to a first 3D eye model modeling corneal refraction, for a plurality of given values of at least one eye state variable, calculate, using a given algorithm, the at least one eye state variable making use of one or more of the synthetic images and a further 3D eye model having at least one parameter, determine a characteristic of a pupil image (image of the pupil) within each of the synthetic images, determine one or more hypothetically optimal values of the at least one parameter of the further 3D eye model that minimize the error between the value(s) of the at least one given eye state variable and the value(s) of the corresponding eye state variable obtained when applying the given algorithm, and establish a relationship between the one or more hypothetically optimal values of the at least one parameter of the further 3D eye model and the characteristic of the pupil image. The relationship can be stored in a memory.

According to an embodiment of a system for determining at least one eye state variable of at least one eye of a subject, the eye comprising an eyeball, an iris defining a pupil, and a cornea, the at least one eye state variable being derivable from at least one image of the eye taken with a camera of known camera intrinsics, the system comprises a device comprising at least one camera of known camera intrinsics for producing image data including at least one eye of a subject, the at least one camera comprising a sensor defining an image plane, the at least one eye comprising an eyeball, an iris defining a pupil, and a cornea. The system further comprises a computing and control unit configured to receive image data of the at least one eye from the at least one camera, determine a characteristic of the image of the pupil within the image data, calculate, using a given algorithm, the at least one eye state variable making use of the image data and a 3D eye model having at least one parameter, the parameter depending in a pre-determined relationship on the characteristic, the relationship being retrieved from a memory.

Other embodiments include (non-volatile) computer-readable storage media or devices, and one or more computer programs recorded on one or more computer-readable storage media or computer storage devices. The one or more computer programs can be configured to perform particular operations or processes by virtue of including instructions that, when executed by one or more processors of a system, in particular one of the systems as explained herein, cause the system to perform the operations or processes.

## BRIEF DESCRIPTION OF THE DRAWINGS

The components in the figures are not necessarily to scale, instead emphasis being placed upon illustrating the principles of the invention. Moreover, in the figures, like reference numerals designate corresponding parts. In the drawings:

Figs. 1A-1C illustrate a top, front and lateral views of a device according to an example;
Figs. 2A-2C and 3 illustrate geometry used in example algorithms suitable for determining eye state variables;
Fig. 4A is a schematic view of an exemplary two-sphere 3D eye model which models corneal refraction;
Figs. 4B-4C illustrate examples of synthetic images obtainable based on a 3D eye model such as the one of Fig. 4A under use of different sets of eye state variables;
Figs. 5A, 6A and 6B show geometric concepts illustrating basic ideas of embodiments;
Figs. 5B and 5C illustrate the effectiveness of adaptation of a parameter of a 3D eye model for generating data for use in determining an eye state variable according embodiments;
Figs. 7 and 7B illustrate flow charts of methods according to embodiments.

## DETAILED DESCRIPTION

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with reference to the orientation of the Figure(s) being described. Because components of embodiments can be positioned in a number of different orientations, the directional terminology is used for purposes of illustration and is in no way limiting. It is to be understood that other embodiments may be utilized and structural or logical changes may be made without departing from the scope of the present invention. The following detailed description, therefore, is not to be taken in a limiting sense, and the scope of the present invention is defined by the appended claims.

The terms "user" and "subject" are used interchangeably and designate a human or animal being having one or more eyes.

The term "3D" is used to signify "three-dimensional".

The terms "eye state" and "eye state variable(s)" are used to signify quantities that characterize the pose of an eye (e.g. eyeball position and orientation such as via the gaze vector in a given coordinate system), the size of the pupil or any other quantity that is typically primarily variable during observation of a real eye. In contrast, the term "eye model parameter(s)" is used to signify quantities which characterize an abstract, idealized 3D model of an eye, e.g. a radius of an eyeball, a radius of an eye sphere, a radius (of curvature) of a cornea, an outer radius of an iris, an index of refraction of certain eye structures or various distance measures between an eyeball center, a pupil center, a cornea center, etc. Statistical information about such parameters like their means and standard deviations can be measured for a given species, like humans, for which such information is typically known from the literature.

It is a task of the invention to provide methods and systems allowing for improved generating, in particular computationally faster, easier and/or more reliably and accurately generating of data suitable for determining eye state variables of a human or animal eye, and correspondingly to provide methods, systems and devices for determining such eye state variables. A suitable device includes one or more cameras for generating image data of one or more respective eyes of a human or animal subject or user within the field-of-view of the device.

Said task is solved by the subject matter of the independent claims.

The device may be a head-wearable device, configured for being wearable on a user's head and may be used for determining one or more gaze- and/or eye-related state variables of a user wearing the head-wearable device.

Alternatively, the device may be remote from the subject, such as a commonly known remote eye-tracking camera module.

The head-wearable device may be implemented as a (head-wearable) spectacles device comprising a spectacles body, which is configured such that it can be worn on a head of a user, for example in a way usual glasses are worn. Hence, the spectacles device when worn by a user may in particular be supported at least partially by a nose area of the user's face. The head-wearable device may also be implemented as an augmented reality (AR-) and/or virtual reality (VR-) device (AR/VR headset), in particular a goggles, or a head-mounted display (HMD). For the sake of clarity, devices are mainly described with regard to head-wearable spectacles devices in the following.

The device has at least one camera having a sensor arranged in or defining an image plane for producing image data, typically taking images, of one or more eyes of the user, e.g. of a left and/or a right eye of the user. In other words, the camera, which is in the following also referred to as eye camera, may be a single camera of the device. This may in particular be the case if the device is remote from the user. As used herein, the term "remote" shall describe distances of approximately more than 20 centimeters from the eye(s) of the user. In such a setup, a single eye camera may be able to produce image data of more than one eye of the user simultaneously, in particular images which show both a left and right eye of a user.

Alternatively, the device may have more than one eye camera. This may in particular be the case if the device is a head-wearable device. Such devices are located in close proximity to the user when in use. An eye camera located on such a device may thus only be able to view and image one eye of the user. Such a camera is often referred to as near-eye camera. Typically, head-wearable devices thus comprise more than one (near-)eye camera, for example, in a binocular setup, at least a first or left (side) eye camera and a second or right (side) eye camera, wherein the left camera serves for taking a left image or a stream of images of at least a portion of the left eye of the user, and wherein the right camera takes an image or a stream of images of at least a portion of a right eye of the user. In the following any eye camera in excess of 1 is also called further eye camera.

In case of a head-wearable device, the eye camera(s) can be arranged at the spectacles body in inner eye camera placement zones and/or in outer eye camera placement zones, in particular wherein said zones are determined such that an appropriate picture of at least a portion of the respective eye can be taken for the purpose of determining one or more eye state variables. In particular, the cameras may be arranged in a nose bridge portion and/or in a lateral edge portion of the spectacles frame, such that an optical field of a respective eye is not obstructed by the respective camera. For example, the cameras can be integrated into a frame of the spectacles body and thereby being non-obstructive.

Furthermore, the device may have illumination means for illuminating the left and/or right eye of the user, in order to increase image data quality, in particular if the light conditions within an environment of the spectacles device are not optimal. Infrared (IR) light may be used for this purpose. Accordingly, the recorded eye image data does not necessarily need to be in the form of pictures as visible to the human eye, but can also be an appropriate representation of the recorded (filmed) eye(s) in a range of light non-visible for humans.

The eye camera(s) is/are typically of known camera intrinsics. As used herein, the term "camera of known camera intrinsics" shall describe that the optical properties of the camera, in particular the its imaging properties are known and/or can be modeled using a respective camera model including the known intrinsic(s) (parameters) approximating the eye camera producing the eye images. Typically, a pinhole camera model is used and full perspective projection is assumed for modeling the eye camera and imaging process. The known intrinsic parameters may include a focal length of the camera, an image sensor format of the camera, a principal point of the camera, a shift of a central image pixel of the camera, a shear parameter of the camera, and/or one or more distortion parameters of the camera.

The eye state of the subject's eye typically refers to an eyeball, a gaze and/or a pupil of the subject's eye, in particular it may refer to and/or be a center of the eyeball, in particular a center of rotation of the eyeball or an optical center of the eyeball, or a certain subset of 3D space in which said center is to be located, like for example a line in 3D, or a gaze-related variable of the eye, for example a gaze direction, a cyclopean gaze direction, a 3D gaze point, a 2D gaze point, a visual axis orientation, an optical axis orientation, a pupil axis orientation, a line of sight orientation, a limbus major and/or minor axes orientation, an eye cyclo-torsion, an eye vergence, a statistics over eye adduction and/or eye abduction, and a statistics over eye elevation and/or eye depression, and data about drowsiness and/or awareness of the user.

The eye state (variable(s)) may as well refer to and/or be a measure of the pupil size of the eye, such as a pupil radius, a pupil diameter or a pupil area.

Gaze- or eye-related variables, points and directions are typically determined with respect to a coordinate system that is fixed to the eye camera(s) and/or the device.

For example, (a) Cartesian coordinate system(s) defined by the image plane(s) of the eye camera(s) may be used.

Variables, points and directions may also be specified or determined within and/or converted into a device coordinate system, a head coordinate system, a world coordinate system or any other suitable coordinate system.

In particular, if the device comprises more than one eye camera and the relative poses, i.e. the relative positions and orientations of the eye cameras, are known, geometric quantities like points and directions which have been specified or determined in any one of the eye camera coordinate systems can be converted into a common coordinate system. Relative camera poses may be known because they are fixed by design, or because they have been measured after each camera has been adjusted into it's use position.

Eye model parameter(s) may for example be a distance between a center of an eyeball, in particular a rotational, geometrical or optical center, and a center of a pupil or cornea, a size measure of an eyeball, a cornea or an iris such as an eyeball radius, a cornea radius, an iris diameter, a distance pupil-center to cornea-center, a distance cornea-center to eyeball-center, a distance pupil-center to limbus center, a distance crystalline lens to eyeball-center, to cornea center and/or to corneal apex, a refractive property of an eye structure such as an index of refraction of a cornea, vitreous humor or crystalline lens, an ellipsoidal shape measure of an eyeball or cornea, a degree of astigmatism, and an eye intra-ocular distance or inter-pupillary distance.

In the following, exemplary algorithms for determining eye state variables using 3D eye models will be discussed. Other such algorithms exist and can be used in the methods, devices and systems of the present invention.

In one example, an algorithm suitable for determining eye state variables of at least one eye of a subject, the eye comprising an eyeball and an iris defining a pupil, includes receiving image data of an eye at a first time from a camera of known camera intrinsics, which camera defines an image plane. A first ellipse representing a border of the pupil of the eye at the first time is determined in the image data. The camera intrinsics and the first ellipse are used to determine a 3D orientation vector of a first circle in 3D and a first center line on which a center of the first circle is located in 3D, so that a projection of the first circle, in a direction parallel to the first center line, onto the image plane is expected to reproduce the first ellipse. A first eye intersecting line in 3D expected to intersect a 3D center of the eyeball at the first time is determined as a line which is, in the direction of the orientation vector, parallel-shifted to the first center line by an expected distance between the center of the eyeball and a center of the pupil.

Accordingly, the first eye intersecting line, which limits the position of the center of the eyeball to a line and thus can be considered as one of several variables characterizing the state of the eye, can be determined without using glints or markers and with low calculation costs, low numerical effort and/or very fast. This even allows determining the state of an eye in real time (within sub-milliseconds range per processed image) with comparatively low hardware requirements. Accordingly, eye state variables may be determined with hardware that is integrated into a head-wearable device during taking eye images with the camera of the head-wearable device and with a negligible delay only, respectively with hardware of low computational power, like smart devices, connectable to the head-wearable device.

Note that the process of determining the orientation vector of the first circle and the first center line is typically done similar as explained in reference [1]. Reference [1] describes a method of 3D eye model fitting and gaze estimation based on pupil shape derived from (monocular) eye images. Starting from a camera image of an eye and having determined the area of the pupil represented by an ellipse, the first step is to determine the circle in 3D space, which gives rise to the observed elliptical image pupil, assuming a (full) perspective projection and known camera parameters. Once this circle is found, it can serve as an approximation of the actual pupil of the eye, i.e. the approximately circular opening of varying size within the iris.

Note that due to a property of perspective projection, the circle center line, i.e. the line in 3D on which lie the centers of all possible circles which produce one and the same particular ellipse in 2D (camera) image space under perspective projection, is NOT trivially obtained, since it does NOT go through the center of said ellipse. Instead, the rather involved mathematical methods for achieving this "unprojection" are explained in reference [3].

Said ellipse "unprojection" as detailed in [3] gives rise to two ambiguities: firstly, there are two solution circles for a given fixed circle radius on the cone which represents the space of all possible solutions. Deciding which one is a correct pupil candidate is described in [1].

The second ambiguity is a size-distance ambiguity, which is the harder one to resolve: given only a 2D image of the pupil it is not possible to know a priori whether the pupil is small and close to the camera or large and far away from the camera. This second ambiguity is resolved in reference [1] by generating a model which comprises 3+3N parameters, including the 3 eyeball center coordinates and parameters of pupil candidates extracted from a time series of N camera images. This model is then optimized numerically in a sophisticated iterative fashion to yield the final eyeball center coordinates.

Note that even the projection of the 3D eyeball center into 2D image space can in general NOT be trivially obtained based on 2D pupil image measures or calculations alone. Some prior art alleges that this can be done by intersecting minor axes of multiple pupil image ellipse observations under varying gaze angles. This is true if and only if the optical axis of the camera points exactly at the 3D eyeball center, which is in general NOT the case, respectively can NOT be reliably achieved or assumed when acquiring images of real eyes. Even IF such a 2D projection of the true eyeball center could be obtained, the mere derivation of such a point in 2D image space does firstly not imply the construction of a line in 3D from the camera through said point and secondly, even IF such a line would be constructed, it wouldn't resolve the size-distance ambiguity. This is the reason why reference [1] resorts to iterative numerical optimization as explained above.

Note also, that algorithms exist which make use of the OUTER iris contour or limbus, i.e. the edge or contour where the iris and cornea transition to the sclera. They have the advantage that unlike the pupil (the "inner" iris contour), the outer iris contour does not change size. Furthermore, in humans the outer iris has a fairly uniform radius of $r_i = 6mm$, and is often used as a parameter of a 3D eye model. When detecting the outer iris contour as an elliptical shape in a camera image, it is thus possible to apply the same strategies as outlined in [1] and [3] and calculate the circle in 3D that gave rise to said elliptical shape - the size-distance ambiguity does not exist in this case, since the size of the circle in 3D can be assumed as known. However, limbus tracking methods have two inherent disadvantages. Firstly, the contrast of the limbus is mostly inferior to the contrast of the pupil, and secondly, larger parts of the limbus are usually occluded, either by the eye lids or - in particular if head-mounted eye cameras are used - because the viewing angle of the camera onto the eye makes it difficult or impossible to image the entire iris. Both issues make reliable limbus detection difficult and pupil detection based methods for determining eye state variables are thus largely preferable, in particular in head-mounted scenarios using near-eye cameras.

Compared to reference [1], the solutions proposed in [4], WO2020/244752 and WO2020/244971 for resolving the size-distance ambiguity (which is based on the proposed eye intersecting line(s)) represents a considerable conceptual and computational simplification. This allows determining eye state variables such as eyeball position(s), pupil size(s) and gaze direction(s) in real-time with considerably reduced hardware and/or software requirements. Accordingly, lightweight and/or comparatively simple head-wearable devices may be more broadly used for purposes like gaze-estimation and/or pupillometry, i.e. measuring the actual size of the pupil in physical units of length.

Note that these methods do not require taking into account a glint from the eye for generating data suitable for determining eye state variables. In other words, the methods are glint-free and do not require using structured light and/or special purpose illumination hardware.

Note further that eyes within a given species, e.g. humans, only vary in size within a very narrow margin and many physiological parameters can thus be assumed constant/equal between different subjects, which enables the use of 3D models of an average eye for the purpose of determining eye state variables. An example for such a physiological parameter is the distance R between center of the eyeball, in the following also referred to as eyeball center, and center of the pupil, in the following also referred to as pupil center. In human eyes the distance R can be assumed with high accuracy as a constant (R = 10.39 mm), which can therefore be used as the expected distance in a 3D model of the human eye for calculating eye state variables.

Therefore, the expected value R can be used to construct an ensemble of possible eyeball center positions (a 3D eye intersecting line), based on an ensemble of possible pupil center positions (a 3D circle center line) and a 3D orientation vector of the ensemble of possible 3D pupil circles, by parallel-shifting the 3D circle center line by the expected distance R between the center of the eyeball and a center of the pupil along the direction of the 3D orientation vector. Note again that in this particular scenario, distance R is a (constant) physiological parameter of the underlying 3D eye model and NOT a quantity that needs to be measured for each subject.

Each further image / observation of one and the same eye but with a different gaze direction gives rise to an independent eye intersecting line in 3D. Finding the nearest point between or intersection of at least two independent eye intersecting lines thus yields the coordinates of the eyeball center in a non-iterative manner. This provides considerable conceptual and computational simplification over prior art methods.

Accordingly, in a *monocular* version of an example algorithm for determining eye state variables, this algorithm includes receiving a second image of the eye at a second time from the camera, more typically a plurality of further images at respective times, determining a second ellipse in the second image, the second ellipse at least substantially representing the border of the pupil at the second time, more typically determining for each of the further images a respective ellipse, using the second ellipse to determine an orientation vector of a second circle and a second center line on which a center of the second circle is located, so that a projection of the second circle, in a direction parallel to the second center line, onto the image plane is expected to reproduce the second ellipse, more typically using the respective ellipse to determine an orientation vector of the further circle and a further center line on which the further circle is located, so that a projection of the further circle, in a direction parallel to the further center line, onto the image plane is expected to reproduce the respective further ellipse, and determining a second eye intersecting line expected to intersect the center of the eyeball at the second time as a line which is, in the direction of the orientation vector of the second circle, parallel-shifted to the second center line by the expected distance, more typically determining further eye intersecting lines each of which is expected to intersect the center of the eyeball at the respective further time as a line which is, in the direction of the orientation vector of the further circle, parallel-shifted to the further center line by the expected distance.

In other words, a camera model such as a pinhole camera model describing the imaging characteristics of the camera and defining an image plane (and known camera intrinsic parameters as parameters of the camera model) is used to determine for several images taken at different times with the camera an orientation vector of a respective circle and a respective center line on which a center of the circle is located, so that a projection of the circle, in a direction parallel to the center line, onto the image plane reproduces the respective ellipse in the camera model, and determining a respective line which is, in the direction of the orientation vector, which typically points away from the camera, parallel-shifted to the center line by an expected distance between a center of an eyeball of the eye and a center of a pupil of the eye as an eye intersecting line which intersects the center of the eyeball at the corresponding time. Thereafter, the eyeball center may be determined as nearest intersection point of the eye intersecting lines in a least squares sense.

Typically, the respective images of the eye which are used to determine the plurality of eye intersecting lines ($D_k$, k = 1 ... n) are acquired with a frame rate of at least 25 frames per second (fps), more typical of at least 30 fps, more typical of at least 60 fps, and more typical of at least 120 fps or even 200 fps.

Once the eyeball center is known, other eye state variables of the human eye such as gaze direction and pupil radius or size can also be calculated non-iteratively.

In particular, an expected gaze direction of the eye may be determined as a vector which is antiparallel to the respective circle orientation vector.

Further, the expected co-ordinates of the center of the eyeball may be used to determine for at least one of the times an expected optical axis of the eye, an expected orientation of the eye, an expected visual axis of the eye, an expected size of the pupil and/or an expected radius of the pupil.

Furthermore, at one or more later times a respective later image of the eye may be acquired by the camera and used to determine, based on the determined respective later eye intersecting line, at the later time(s) an expected gaze direction, an expected optical axis of the eye, an expected orientation of the eye, an expected visual axis of the eye, an expected size of the pupil and/or an expected radius of the pupil.

In such a monocular version of an example algorithm for determining eye state variables, the need remains to acquire a time series of N>1 eye images (also called observations) and the method requires those observations to show the eye under a relatively large variation of gaze angles in order for the intersection of those N eye intersecting lines to provide a reliable eyeball center calculation.

Accordingly, in another, *binocular* version of an example algorithm for determining eye state variables of one or more eyes, this algorithm includes receiving image data of a further eye of the subject at a second time, substantially corresponding to the first time, from a camera of known camera intrinsics and defining an image plane, the further eye comprising a further eyeball and a further iris defining a further pupil, determining a further ellipse in the image data, the further ellipse at least substantially representing the border of the further pupil of the further eye at the second time, using the camera intrinsics and the further ellipse to determine a 3D orientation vector of a further circle in 3D and a further center line on which a center of the further circle is located in 3D, so that a projection of the further circle, in a direction parallel to the further center line, onto the image plane is expected to reproduce the further ellipse, and determining a further eye intersecting line in 3D expected to intersect a 3D center of the further eyeball at the second time as a line which is, in the direction of the 3D orientation vector of the further circle, parallel-shifted to the further center line by an expected distance between the center of the further eyeball and a center of the further pupil.

In other words, instead of a purely *monocular* paradigm, image data from more than one eye of the subject, recorded substantially simultaneously can be leveraged in a *binocular* or multiocular setup.

Typically, the respective images of an/each eye which are used to determine the eye intersecting lines are acquired with a frame rate of at least 25 frames per second (fps), more typical of at least 30 fps, more typical of at least 60 fps, and more typical of at least 120 fps or even 200 fps.

In this way, in case image data from one eye originates from a different eye camera than image data from a further eye, it can be guaranteed that eye observations are sufficiently densely sampled in time in order to provide substantial simultaneous image data of different eyes. Image frames stemming from different cameras can be marked with timestamps from a common clock. This way, for each image frame recorded by a given camera at a (first) time t, a correspondingly closest image frame recorded by another camera at a (second) time t' can be selected, such that abs(t-t') is minimal (e.g. at most 2.5 ms if cameras capture image frames at 200 fps).

In case image data from one eye and from a further eye originates from the same camera, the second time can naturally correspond exactly to the first time, in particular the image data of the eye and the image data of the further eye can be one and the same image comprising both (all) eyes.

Such a binocular algorithm may include using the first eye intersecting line and the further eye intersecting line to determine expected coordinates of the center of the eyeball and of the center of the further eyeball, such that each eyeball center lies on the respective eye intersecting line and the 3D distance between the eyeball centers corresponds to a predetermined value (IED, IPD), in particular a predetermined inter-eyeball or inter-pupillary distance.

Accordingly, the centers of both eyeballs of a subject may be determined simultaneously, based on a binocular observation at merely a single point in time, instead of having to accumulate a time series of N>1 observations. Also, no monocular intersection of eye intersecting lines needs to be performed and this algorithm thus works under entirely static gaze of the subject, on a frame by frame basis. This is made possible by the insight that the distance between two eyes of a subject can be considered another physiological constant and can thus be leveraged for determining eye state variables of one or more eyes of a subject in the framework of an extended 3D eye model.

The predetermined distance value (IED, IPD) between the center of the eyeball and the center of the further eyeball can be an average value, in particular a physiological constant or population average, or an individually measured or known value of the subject. The average human inter-pupillary distance (IPD) at fixation at infinity can be assumed as IPD = 63.0mm. This value is therefore a proxy for the actual 3D distance between the eyeball centers of a subject, the inter-eyeball distance (IED). Individually measuring the IPD can for example be performed with a simple ruler, as routinely done by optometrists.

The expected coordinates of the center of the eyeball and of the center of the further eyeball can in particular be determined, such that the radius of the first circle in 3D, representing the pupil of the eyeball, and the radius of the further circle in 3D, representing the further pupil, are substantially equal. As a further insight, it is possible to leverage the physiological fact that in most beings, pupils of different eyes are controlled by the same neural pathways and can not change size independently of each other. In other words, the pupil size of the left and of the right eye of for example a human is substantially equal at any instant in time.

Mathematically requesting the condition that the size of the circle and the size of the further circle in 3D have to be equal provides an unambiguous solution which yields both 3D eyeball center positions as well as the pupil size with merely a single binocular observation in time.

This non-iterative method is numerically stable, especially under static gaze conditions, and extremely fast and can be performed on a frame by frame basis in real-time. Alternatively, to be more robust to noise, observations can be averaged over a given time span. Once the center of an eyeball is known, other eye state variables such as an expected gaze direction, optical axis, orientation, visual axis of the eye, size or radius of the pupil of the eye can be calculated (also non-iteratively) for subsequent observations at later instants in time, simply based on the "unprojection" of pupil ellipse contours, providing even faster computation.

The algorithms detailed above merely constitute examples of algorithms for determining eye state variables, which make use of a 3D eye model. Other such algorithms are possible and can be used in the methods according to the invention.

According to the invention and contrary to prior art methods, effects of refraction by the cornea may be taken into account by adapting the 3D eye model.

It has been surprisingly found, that the simple cornea-less 3D eye model employed in [1], and which forms the basis of calculating approximate eye state variables in [4], WO2020/244752 and WO2020/244971, can be adapted to yield the correct eye state values at runtime in the following way. Note first that said eye model employed in [1] has a single parameter, namely the (physiologically constant) distance R between eyeball rotation center and pupil center. Note further that the shape and degree of distortion of the pupil image as seen by the eye camera depends in a complex non-linear manner on the pose of the eye with respect to the camera and the radius of the pupil (see reference [5]). The pose of the eye is composed of the orientation of the gaze direction of the eye with respect to the optical axis of the camera and the position of the eyeball with respect to the camera (i.e. in general offset from the optical axis of the camera and at an unknown distance). In fact, even *given* a particular pose of the eye with respect to the camera and *given* the pupil radius, it is impossible to analytically calculate the pupil contour as it would appear in a camera image under perspective projection, due to the complex non-linear nature of refraction through the cornea. This is only possible in scenarios like described in [1], based on an eye model which has no cornea - the perspective projection of a pupil assumed as a perfect circle is then a perfect ellipse in the image, which ellipse can be analytically calculated given a particular set of eye state variables (pose and pupil radius). As soon as an eye with a cornea is considered, no closed-form analytical solution characterizing the shape of the pupil under perspective projection given the pose of the eye and the pupil radius is possible. Likewise, the "inverse" problem, to derive the pose the eye and pupil radius based on the image of the pupil also has no closed-form analytical solution.

It has now been surprisingly found by the inventors, that a quantity which is very easily obtainable from the camera image, namely a measure of the shape which represents the pupil in the camera image, like for example a circularity measure, can not only serve as a first order approximation or "summary" of this eye pose and pupil radius dependent distortion, but at the same time is suitable to make the simple cornea-less 1-parameter eye model adaptive to said measure of shape. In other words, that it is possible to find simple relationships which make the parameter(s) of the 3D eye models of the prior art adaptive to account for the effects of corneal refraction in a very simple and efficient way.

Note that algorithms exist which try to derive or fit an individual value for one or more parameters of a 3D eye model for each subject or even for a particular/each eye, as part of numerical optimization schemes. This however brings the disadvantages of iterative optimization based algorithms like [1], which have already been mentioned. In particular, this has to be done using real world eye image data, i.e. in real-time, which is typically not feasible.

Note further that the goal of the present invention is NOT to determine individual geometrico-morphological measurements of an individual subject. Such measurements can be done offline in a non time critical manner. In general such individual measurements are also often not necessary for more general determination of eye state variables in an eye tracking context, since variation in individual eyeball measures are limited as already mentioned. Employing "average" 3D eye models which represent a certain population of subjects is in many cases a viable strategy to obtain statistically significant results of eye state variables in experiments with multiple subjects, like for example in many pupillometry studies. The present invention therefore provides the advantage of providing "adaptive" eye model parameters, derived via eye models of population averages but correctly modeling corneal refraction, as a function of a pupil image observation characteristics. This way, non time critical offline simulations using eye models aware of corneal refraction can enable calibration-free methods for determining eye state variables in real-time using simpler eye models which are "made" refraction aware via simple pre-established relationships between eye model parameters and easily obtainable pupil image characteristics.

According to an embodiment of a method for generating data suitable for determining at least one eye state variable of at least one eye of a subject, the eye comprising an eyeball, an iris defining a pupil, and a cornea, the at least one eye state variable being derivable from at least one image of the eye taken with a camera of known camera intrinsics, the method includes providing a first 3D eye model modeling corneal refraction. Using the known camera intrinsics, synthetic image data of several model eyes according to the first 3D eye model is generated for a plurality of given values of at least one eye state variable. Using a given algorithm the at least one eye state variable is calculated using one or more of the synthetic images and a further 3D eye model having at least one parameter. A characteristic of the image of the pupil within each of the synthetic images is determined and one or more hypothetically optimal values of the at least one parameter of the further 3D eye model that minimize the error between the value(s) of the at least one given eye state variable and the value(s) of the corresponding eye state variable obtained when applying the given algorithm are determined. Finally, a relationship between the one or more hypothetically optimal values of the at least one parameter of the further 3D eye model and the characteristic of the pupil image is established.

According to an embodiment of a method for determining at least one eye state variable of at least one eye of a subject, the eye comprising an eyeball, an iris defining a pupil, and a cornea, the at least one eye state variable being derivable from at least one image of the eye taken with a camera of known camera intrinsics, the method includes receiving image data of the at least one eye from a camera of known camera intrinsics and defining an image plane. Further, a characteristic of the image of the pupil within the image data is determined. A 3D eye model having at least one parameter is provided, the parameter depending in a pre-determined relationship on the characteristic. Finally, the method further includes using a given algorithm to calculate the at least one eye state variable using the image data and the 3D eye model including the at least one characteristic-dependent parameter.

According to a preferred embodiment of either method, the characteristic of the image of the pupil may be a measure of the circularity of the pupil area or outline, in particular a ratio of minor to major axis length of an ellipse fit to the pupil image area, a measure of variation of the curvature of the pupil outline, a measure of elongation of the pupil or a measure of the bounding box of the pupil area.

Typically, the relationship between the hypothetically optimal values of the at least one further 3D eye model parameter and the characteristic of the pupil image may be a constant value, in particular a constant value smaller or larger than the corresponding average parameter of the first 3D eye model, a linear relationship, or a polynomial relationship, or another non-linear relationship, e.g. based on a regression fit. This relationship may be stored to/in a memory. That way, a given algorithm to calculate the at least one eye state variable using the image data and a 3D eye model including the at least one parameter may later retrieve the relationship from memory and use it to calculate the one or more eye state variables in a fast and accurate way, taking corneal refraction into account, by making use of a pupil characteristic-dependent 3D eye model parameter.

In a particular embodiment, the 3D eye model respectively the further 3D eye model has at most one parameter. In particular, unlike the first 3D eye model, they do not need to model corneal refraction. Thus a very simple and fast method is provided.

Alternatively, the 3D eye model respectively the further 3D eye model may have more than one parameter and in a variant a separate relationship may be established for more than one of them with the pupil characteristic. In this way, the advantages of more complex eye models may be leveraged.

The further 3D eye model of the embodiments of methods for generating data suitable for determining at least one eye state variable and the 3D eye model of embodiments of methods for determining at least one eye state variable may be the same model, or may be partly different, the only decisive point being that they comprise a corresponding parameter for which a relationship with the characteristic of the pupil has been established.

Examples for parameters of the (any) 3D eye model as described in embodiments are a distance between a center of an eyeball, in particular a rotational, geometrical or optical center, and a center of a pupil or cornea, a size measure of an eyeball, a cornea or an iris such as an eyeball radius, a cornea radius, an iris diameter, a distance pupil-center to cornea-center, a distance cornea-center to eyeball-center, a distance pupil-center to limbus center, a distance crystalline lens to eyeball-center, to cornea center and/or to corneal apex, a refractive property of an eye structure such as an index of refraction of a cornea, vitreous humor or crystalline lens, an ellipsoidal shape measure of an eyeball or cornea, a degree of astigmatism, and an eye intra-ocular distance or inter-pupillary distance.

According to a variant, said relationship between a particular 3D eye model parameter and the characteristic of the pupil may be the same for all eye state variables.

According to a preferred embodiment, a different relationship between a parameter of the 3D eye model respectively the further 3D eye model and the characteristic of the pupil image may be/have been established for each eye state variable or for groups of eye state variables. This way, different ways in which a certain parameter of a 3D eye model influences the determination of certain eye state variable as a suite of the particular given algorithm used can be taken into account, and an optimal accuracy for all eye state variables of interest can be achieved.

The eye state variable typically is selected from the list of a pose of an eye such as a location of an eye, in particular an eyeball center, and/or an orientation of an eye, in particular a gaze vector, optical axis orientation or visual axis orientation, a 3D circle center line, a 3D eye intersecting line, and a size measure of a pupil of an eye, such as a pupil radius or diameter.

Further, the given algorithm typically does not take into account a glint from the eye for calculating the at least one eye state variable, in other words the algorithm is "glint-free". Also, the algorithm typically does not require structured light and/or special purpose illumination to derive eye state variables.

The given algorithm typically calculates the at least one eye state variable in a non-iterative way.

According to an embodiment, a system for generating data suitable for determining at least one eye state variable of at least one eye of a subject, the eye comprising an eyeball, an iris defining a pupil, and a cornea, the at least one eye state variable being derivable from at least one image of the eye taken with a camera of known camera intrinsics is provided. The system comprising a computing and control unit configured to generate, using the known camera intrinsics, synthetic image data of several model eyes according to a first 3D eye model modeling corneal refraction, for a plurality of given values of at least one eye state variable, to calculate, using a given algorithm, the at least one eye state variable making use of one or more of the synthetic images and a further 3D eye model having at least one parameter, to determine a characteristic of the image of the pupil within each of the synthetic images, to determine one or more hypothetically optimal values of the at least one parameter of the further 3D eye model that minimize the error between the value(s) of the at least one given eye state variable and the value(s) of the corresponding eye state variable obtained when applying the given algorithm, and to establish a relationship between the one or more hypothetically optimal values of the at least one parameter of the further 3D eye model and the characteristic of the pupil image and store it in a memory.

Typically, the computing and control unit is configured to perform the methods for generating data suitable for determining at least one eye state variable of at least one eye of a subject as explained herein.

The computing and control unit of the system may be part of a device such as a personal computer, laptop, server or part of a cloud computing system.

According to an embodiment, a system for determining at least one eye state variable of at least one eye of a subject, the eye comprising an eyeball, an iris defining a pupil, and a cornea, the at least one eye state variable being derivable from at least one image of the eye taken with a camera of known camera intrinsics is provided. The system comprises a device comprising at least one camera of known camera intrinsics for producing image data including at least one eye of a subject, the at least one camera comprising a sensor defining an image plane, the at least one eye comprising an eyeball, an iris defining a pupil, and a cornea. The system further comprises a computing and control unit configured to receive image data of the at least one eye from the at least one camera, determine a characteristic of the image of the pupil within the image data, calculate, using a given algorithm, the at least one eye state variable making use of the image data and a 3D eye model having at least one parameter, the parameter depending in a pre-determined relationship on the characteristic, the relationship being retrieved from a memory.

Typically, the computing and control unit of this system is configured to perform the methods for determining at least one eye state variable of at least one eye of a subject as explained herein.

The device may be a head-wearable device or a remote (eye-tracking) device.

The computing and control unit can be at least partly integrated into the device and/or at least partly provided by a companion device of the system, for example a mobile companion device such as a mobile phone, tablet or laptop computer. Both the device and the companion device may have computing and control units, which typically communicate with each other via an interface board (interface controller), for example a USB-hub board (controller). Either of these computing and control units may be solely or partly responsible for determining the one or more eye state variables of an eye and/or a further eye of the user.

Different thereto, as previously mentioned the system for generating data suitable for determining at least one eye state variable of at least one eye of a subject, which generates synthetic image data, may typically comprise a more powerful computing and control unit such as a personal / desktop computer, server or the like. The system for generating data suitable for determining at least one eye state variable of at least one eye of a subject can be connected with or otherwise set into communication with the system for determining at least one eye state variable of at least one eye of a subject, by any suitable means known to the skilled person, in particular to communicate the established relationship(s).

In one embodiment, the head-wearable (spectacles) device is provided with electric power from a companion device of the system during operation of the spectacles device, and may thus not require an internal energy storage such as a battery. Accordingly, the head-wearable (spectacles) device may be particularly lightweight. Further, less heat may be produced during device operation compared to a device with an internal (rechargeable) energy storage. This may also improve comfort of wearing.

The computing and control unit of the head-wearable (spectacles) device may have a USB-hub board, a camera controller board connected with the camera, and a power-IC connected with the camera controller board, the camera and/or the connector for power supply and/or data exchange, and an optional head orientation sensor having an inertial measurement unit (IMU).

Reference will now be made in detail to various embodiments, one or more examples of which are illustrated in the figures. Each example is provided by way of explanation, and is not meant as a limitation of the invention. For example, features illustrated or described as part of one embodiment can be used on or in conjunction with other embodiments to yield yet a further embodiment. It is intended that the present invention includes such modifications and variations. The examples are described using specific language which should not be construed as limiting the scope of the appended claims. The drawings are not scaled and are for illustrative purposes only. For clarity, the same elements or steps have been designated by the same references in the different drawings if not stated otherwise.

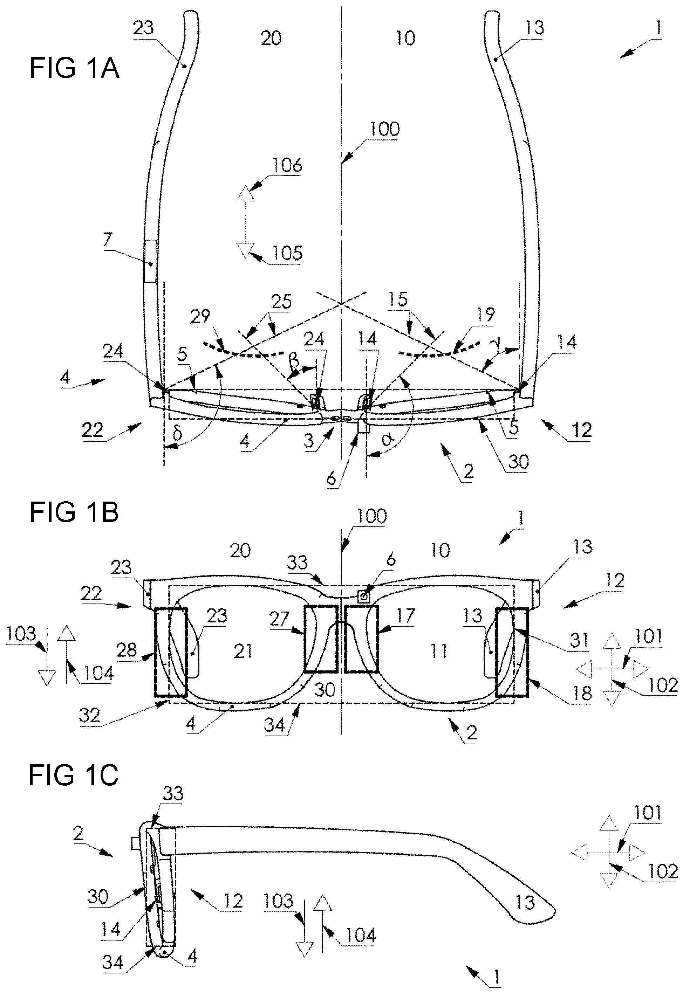

With reference to Figs. 1A to 1C, a generalized example of a head-wearable spectacles device for determining one or more eye state variables of a user is shown. In fact, with the help of Fig. 1A and 1C a plurality of examples shall be represented, wherein said examples mainly differ from each other in the position of the cameras 14, 24. Thus, the spectacles device 1 is depicted in Fig.1A with more than one camera 14, 24 per ocular opening 11, 21 only for presenting each example. However, in this example the spectacles device does not comprise more than one camera 14, 24 associated to each ocular opening 11, 21.

Fig.1A is a view from above on said spectacles device 1, wherein the left side 10 of the spectacles device 1 is shown on the right side of the drawing sheet of Fig.1A and the right side 20 of the spectacles device 1 is depicted on the left side of the drawing sheet of Fig.1A. The spectacles device 1 has a middle plane 100, which coincides with a median plane of the user of the spectacles device 1 when worn according to the intended use of the spectacles device 1. With regard to user's intended use of the spectacles device 1, a horizontal direction 101, a vertical direction 102, 100, a direction "up" 104, a direction "down" 103, direction towards the front 105 and a direction towards the back 106 are defined.

The spectacles device 1 as depicted in Fig.1A, Fig.1B, and Fig.1C comprises a spectacles body 2 having a frame 4, a left holder 13 and a right holder 23. Furthermore, the spectacles body 2 delimits a left ocular opening 11 and a right ocular opening 21, which serve the purpose of providing an optical window for the user to look through, similar to a frame or a body of normal glasses. A nose bridge portion 3 of the spectacles body 2 is arranged between the ocular openings 11, 21. With the help of the left and the right holder 13, 23 and support elements of the nose bridge portion 3 the spectacles device 1 can be supported by ears and a nose of the user. In the following, the frame 4 is also referred to as front frame and spectacles frame, respectively.

According to the examples represented by Fig.1A, a left eye camera 14 and/or a right eye camera 24 can be arranged in the spectacles body 2. Generally, the nose bridge portion 3 or a lateral portion 12 and/or 22 of the spectacles body 2 is a preferred location for arranging/integrating a camera 14, 24, in particular a micro-camera. Different locations of the camera(s) 14, 24 ensuring a good field of view on the respective eye(s) may be chosen. In the following some examples are given.

If a camera 14 or 24 is arranged in the nose bridge portion 3 of the spectacles body 2, the optical axis 15 of the left camera 14 may be inclined with an angle $\alpha$ of 142° to 150°, preferred 144°, measured in counter-clockwise direction (or -30° to -38°, preferred - 36°) with respect to the middle plane 100. Accordingly, the optical axis 25 of the right camera 24 may have an angle $\beta$ of inclination of 30° to 38°, preferred 36°, with respect to the middle plane 100.

If a position of a camera 14, 24 is located in one of the lateral portions 12, 22 of the spectacles body 2, the optical axis 15 of the left camera 14 may have an angle $\gamma$ of 55° to 70°, preferred 62° with respect to the middle plane, and/or the optical axis 25 of the right camera 24 may be inclined about an angle $\delta$ of 125° to 110° (or -55° to -70°), preferred 118° (or -62°).

Furthermore, a bounding cuboid 30 - in particular a rectangular cuboid - may be defined by the optical openings 11, 21, which serves four specifying positions of camera placement zones 17, 27, 18, 28. As shown in Fig.1A, Fig.1B, and Fig.1C the bounding cuboid 30 - represented by a dashed line - may include a volume of both ocular openings 11, 21 and touches the left ocular opening 11 with a left lateral surface 31 from the left side 10, the right ocular opening 21 with a right lateral surface 32 from the right side 20, at least one of the ocular openings 11, 21 with an upper surface 33 from above and from below with a lower surface 34.

In case a left/right camera 14, 24 is arranged in the nose bridge portion 3, a projected position of the left camera 14 would be set in a left inner eye camera placement zone 17 and the right camera 24 would be (projected) in the right inner eye camera placement zone 27.

When being in the left/right lateral portion 12, 22, the left camera 14 may be positioned - when projected in the plane of the camera placement zones - in the left outer eye camera placement zone 18, and the right camera 24 is in the right outer eye camera placement zone 28.

With the help of the front view on the spectacles device 1 depicted in Fig.1B the positions of the eye camera placement zones 17, 18, 27, 28 are explained. In Fig.1B rectangular squares represent said eye camera placement zones 17, 18, 27, 28 in a vertical plane perpendicular to the middle plane 100.

All examples of the spectacles device 1 as represented by Figs. 1A to 1C have in common, that no more than one camera 14/24 is associated to one of the optical openings 11, 21. Typically, the spectacles device 1 does only comprise one or two cameras 14, 24 to produce image data of a left and a right eyeball 19, 29, respectively.

In the example shown in Figs. 1A to 1C, one camera 14 is arranged for producing image data of one eye 19, while the other camera 24 is arranged for producing image data of a further eye 29. By virtue of the precisely known relative poses of cameras 14 and 24, quantities calculated with respect to the 3D coordinate system defined by one camera can be transformed into the 3D coordinate system defined by the other camera or into a common, e.g. headset 3D coordinate system. The same reasoning applies for embodiments with more than two cameras.

The spectacles device 100 as shown in Fig.1A comprises a computing and control unit 7 configured for processing the image data from the left and/or the right camera 14, 24 for determining eye state variables of the respective eye or both eyes.

Typically, the computing and control unit is non-visibly integrated within the holder, for example within the right holder 23 or the left holder 13 of the spectacles device 1. According to a non-shown example, a processing unit can be located within the left holder. Alternatively, the processing of the left and the right images from the cameras 14, 24 for determining the eye state variable(s) may alternatively be performed by a connected companion device such as smartphone or tablet or other computing device such as a desktop or laptop computer, and may also be performed entirely offline, based on videos recorded by the left and/or right cameras 14, 24.

The head wearable device 1 may also include components that allow determining the device orientation in 3D space, accelerometers, GPS functionality and the like.

The head wearable device 1 may further include any kind of power source, such as a replaceable or rechargeable battery, or a solar cell. Alternatively (or in addition), the head wearable device may be supplied with electric power during operation by a connected companion device, and may even be free of a battery or energy source.

The device of the present invention may however also be embodied in configurations other than in the form of spectacles, such as for example as integrated in the nose piece or frame assembly of an AR or VR head-mounted display (HMD) or goggles or similar device, or as a separate nose clip add-on or module for use with such devices. Also, the device may be a remote device, which is not wearable or otherwise in physical contact with the user.

In combination, a device and computing and control unit as detailed above may form a system for determining at least one eye state variable of at least one eye of a subject according to embodiments of the invention.

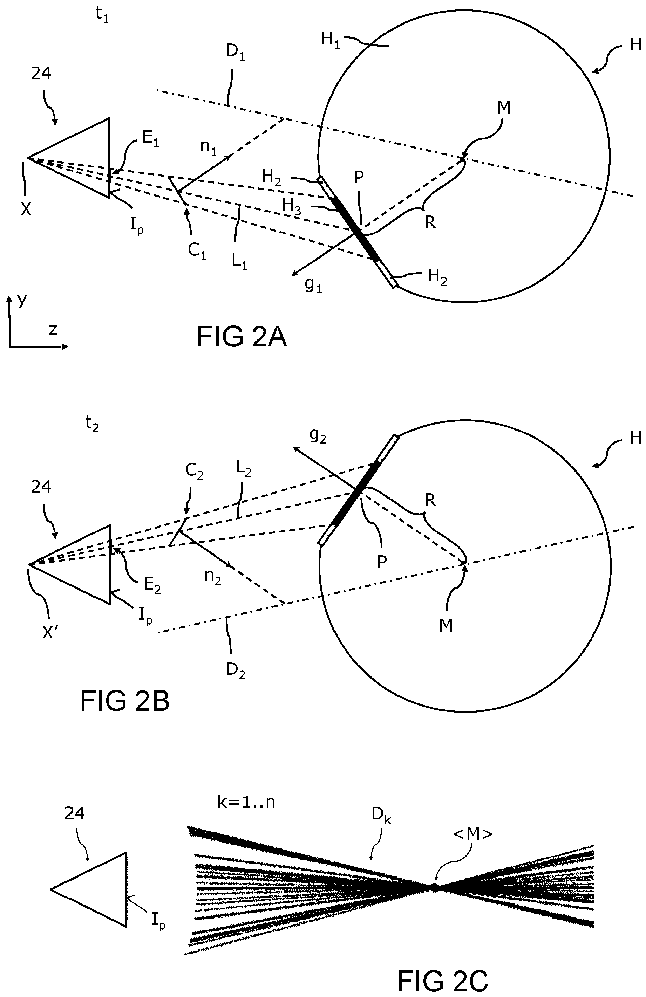

Figs. 2ABC and 3 illustrate geometry used in example algorithms which can be used to calculate eye state variables.

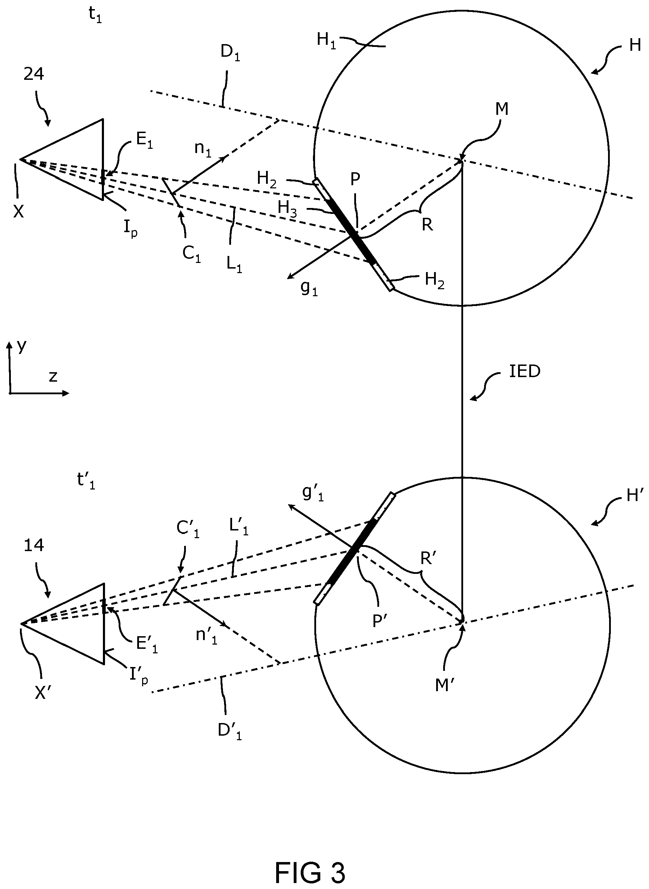

Referring first to the example of Fig.3, the cameras 14 and 24 used for taking images of the user's eyes are modeled as pinhole cameras. The user's eyes H, H' are represented by an appropriate 3D model for human eyes. In particular, the eye model illustrated comprises a single parameter, namely the distance (R,R') between the eyeball center (M,M') and the pupil center (P,P').

Fig.3 illustrates cameras 14, 24 and the human eyes H', H in the respective fields of view of the cameras 14, 24. Determining of (pupil circle) center lines L and eye intersecting lines D will be explained based on one side and camera first (monocularly), the calculations for the other side/camera being analogous. Thereafter, a binocular scenario will be explained. The cameras 14, 24 are typically near-eye cameras as explained above with regard to Figs. 1A-1C. For the sake of clarity, a Cartesian coordinate system y, z is additionally shown (x-axis perpendicular to paper plane). We will assume this to be a common 3D coordinate system into which all quantities originally calculated with respect to an individual camera's coordinate system can be or have been transformed.

In gaze estimation, estimating the optical axis g of the eye is a primary goal. In pupillometry, estimating the actual size (radius) of the pupil in units of physical length (e.g. mm) is the primary goal. The state of the eye model, similar to the one employed by reference [1], is uniquely determined by specifying the position of the eyeball center M and the pose and radius of the pupil $H_3 = (\varphi, \theta, r)$, where $\varphi$ and $\theta$ are the spherical coordinates of the normalized vector pointing from M into the direction of the center of the pupil P. We will refer to $\varphi$ and $\theta$ as gaze angles. In some cases, we will also refer to the angle between the optical axis g and the negative z-axis as gaze angle. To determine the eyeball center M is therefore a necessary first step in video image based, glint-free gaze estimation and pupillometry.

In reference [1], a complex iterative optimization is performed to estimate eyeball positions as well as gaze angles and pupil size based on a time series of observations. In this respect, and in the context of the present disclosure, the expressions "iterative" and "optimization" respectively "optimization based" refer to algorithms which take as input image data from one or several points in time, and try to derive eye state variables in a loop-like application of the same core algorithm, until some cost function or criterion is optimized (e.g. minimized or maximized). Note that the expression "iterative" is thus NOT in any way linked to the fact if the algorithm operates on a single image or on a series of image data from different points in time.

Different thereto, examples of computationally less demanding non-iterative algorithms suitable for use in the methods of the present invention are described in the following. The examples given are based on analytical geometry. However, other non-iterative algorithms which use 3D eye model assumptions in some way may be used. For examples machine-learning based algorithms, like such using neural networks may be combined with 3D eye models.

In particular, as a first step a first ellipse $E_1$ representing a border (outer contour) of the pupil $H_3$ at the first time $t_1$ is determined in a first image taken with the camera 24. This is typically achieved using image processing or machine-learning techniques.

As explained in detail in reference [1] a camera model of the camera 24 is used to determine an orientation vector $n_1$ of the first circle $C_1$ and a first center line $L_1$ on which a center of the first circle $C_1$ is located, so that a projection of the first circle $C_1$, in a direction parallel to the first center line $L_1$, onto the image plane $I_p$ reproduces the first ellipse $E_1$ in the image. In this step, the same disambiguation procedure on pairs of unprojected circles as proposed in reference [1] may be used.

As a result, we obtain circle $C_1$, which we can choose as that circle along the unprojection cone which has radius r = 1.0 mm, and its orientation vector $n_1$ in 3D. We will call $c_1$ the vector from the camera center X (the center of the perspective projection) to the center of this circle $C_1$ of radius r = 1.0 mm, i.e. $c_1 = C_1 - X$. The center line can then be written as $L_1(r) = r*c_1$ with r taking any positive real value. Note that vector $c_1$ does not necessarily have length equal to 1.

However, the size-distance ambiguity explained above remains so far. It is this size-distance ambiguity which is resolved in a much simpler manner than proposed in [1] by the example algorithms presented in the following.

For this purpose, a first eye intersecting line $D_1$ expected to intersect the center M of the eyeball at the first time $t_1$ may be determined as a line which is, in the direction of the orientation vector $n_1$, parallel-shifted to the first center line $L_1$ by the expected distance R between the center M of the eyeball and the center P of the pupil. This expected distance R is usually set to its average human (physiological) value R = 10.39 mm, which is in the following also referred to as a physiological constant of human eyes. In this 3D eye model, this is the sole parameter.

Note that for each choice of pupil radius r, the circle selected by $r*c_1$ constitutes a 3D pupil candidate that is consistent with the observed pupil ellipse $E_1$. In the framework of the 3D eye model, if the circle thus chosen were to be the actual pupil, it would thus need to be tangent to a sphere of radius R and position given by

$$D_1(r) = r * c_1 + R * n_1 \qquad\qquad (Eq.1)$$

defining a line in 3D that is parametrized by pupil radius r, in which $r*c_1$ represents the ensemble of possible pupil circle centers, i.e. the circle center line $L_1$. Note that $n_1$ is normalized to length equal 1, but vector $c_1$ is not, as explained above. As the center of the 3D pupil equals $P = r*c_1$ when r is chosen to be the actual pupil radius, the actual eyeball center M thus indeed needs to be contained in this line $D_1$.

Note again that it is a property of perspective projection, that the center of the ellipse $E_1$ in the camera image, which ellipse is the result of perspective projection of any of the possible 3D pupil circles corresponding to $r*c_1$, does NOT lie on the circle center line $L_1$.

Such eye intersecting lines D and such circle center lines L constitute eye state variables in the sense of the present disclosure.

In a monocular algorithm, referring to Figs. 2A-2C, a second ellipse $E_2$ representing the border of the pupil $H_3$ at a second time $t_2$ can be determined in a second image taken with the camera 24. Likewise, the camera model may be used to determine an orientation vector $n_2$ of the second circle $C_2$ and a second center line $L_2$ on which a center of the second circle $C_2$ is located, so that a projection of the second circle $C_2$, in a direction parallel to the second center line $L_2$, onto the image plane $I_p$ of the camera reproduce the second ellipse $E_2$ in the image. Likewise, a second eye intersecting line $D_2$ expected to intersect the center M of the eyeball at the second time $t_2$ may be determined as a line which is, in the direction of the orientation vector $n_2$, parallel-shifted to the second center line $L_2$ by the distance R. Therefore, since the center M of the eyeball has to be contained in both $D_1$ and $D_2$, it may be determined as intersection point of the first eye intersecting line $D_1$ and the second eye intersecting line $D_2$ or as nearest point to the first eye intersecting line $D_1$ and the second eye intersecting line $D_2$. Note that each unprojection circle $C_k$ constrains the eyeball position M in 3D to the respective line $D_k$ (the subscript k indicates a time or observation index, with k=1 in Fig.2A and k=2 in Fig.2B). Typically, the eye intersecting lines $D_k$ may be determined for a plurality of different times $t_k$ with k = 1 ... n, see Fig.2C. Each of the eye intersecting lines $D_k$ is expected to intersect the center M of the eyeball at respective times. Therefore, the center M of the eyeball is typically determined as nearest point <M> to the eye intersecting lines $D_k$, k = 1 ... n. In other words, in this monocular algorithm, the center M of the eyeball intersection is in practice typically taken in a least-squares sense. After determining the eyeball position M, gaze directions $g_1$, $g_2$ may be determined as being negative to the respective orientation vector $n_1$, $n_2$. The pupil radius r for each observation k can simply be obtained by scaling $r*c_k$ such that the resulting circle is tangent to the sphere centered at M and having radius R. Further, the respective optical axis may be determined as by (normalized) direction from P to the intersection point M.

The number of pupils (image frames) that can be calculated with the monocular algorithm explained above is, for the same computing hardware, typically at least one order of magnitude higher compared to the method of reference [1].

In an example of a binocular algorithm, referring again to Fig.3, the same procedure for generating a 3D circle center line and a 3D eye intersecting line as explained for eyeball H with center M based on image data from camera 24 can be applied to a further eye H' with center M', based on image data from camera 14, at a second time ($t'_1$), substantially corresponding to the first time ($t_1$), yielding corresponding quantities for the further eye, which are denoted with a prime (') in the figure.

The expected distance R' between the center of the eyeball M' and the center of the pupil P' of the further eye H' may be set equal to the corresponding value R of eye H, or may be an eye-specific value.

In an example, a binocular algorithm further comprises using the first eye intersecting line $D_1$ and the further eye intersecting line $D'_1$ to determine expected coordinates of the center M of the eyeball H and of the center M' of the further eyeball H', such that each eyeball center lies on the respective eye intersecting line and the 3D distance between the eyeball centers corresponds to a predetermined value (IED, IPD), in particular a predetermined inter-eyeball distance IED, as indicated in Fig.3. This algorithm is based on the insight that the distance between two eyes of a grown subject changes very little if at all over time and can thus be entered into the method as a further physiological constraint, thus narrowing down the space of possible solutions for finding eye state variables, such as the 3D eyeball centers.

In particular, the predetermined distance value (IED, IPD) between the center of the eyeball and the center of the further eyeball may be an average value, in particular a physiological constant or population average, or an individually measured value of the subject. The average human inter-pupillary distance (IPD) at fixation at infinity can be assumed as IPD=63.0mm. This value is therefore a proxy for the actual 3D distance between the eyeball centers of a human subject, the inter-eyeball distance (IED). Individually measuring the IPD can for example be performed with a simple ruler.

In this example, the center of the eyeball and the center of the further eyeball can for example be found based on some assumption about the geometric setup of the device with respect to the eyes and head of the subject, for example that the interaural axis has to be perpendicular to some particular direction, like for example the z-axis of a device coordinate system such as shown in the example of Fig.3.

In a further example, a binocular algorithm further comprises determining the expected coordinates of the center M of the eyeball and of the center M' of the further eyeball, such that the radius r of the first circle in 3D and the radius r' of the further circle in 3D are substantially equal, thereby also determining said radius. As previously set out, the center of the 3D pupil equals $P = r*c_1$ when r is chosen to be the actual pupil radius. The same applies to the further eye, where $P' = r'*c'_1$, with $c'_1$ being the vector from the camera center X' to the center of this circle $C'_1$ of radius r = 1.0 mm, i.e. $c'_1 = C'_1 - X'$.

As a physiological fact, in most beings pupils of different eyes are controlled by the same neural pathways and can not change size independently of each other. In other words, the pupil size of the left and of the right eye of for example a human is substantially equal at any instant in time. This insight was surprisingly found to enable a particularly simple and fast solution to both the gaze-estimation (3D eyeball center and optical axis) and pupillometry (pupil size) problems, in a glint-free scenario based on a single observation in time of two eyes as follows. Since the center coordinates of the eyeball can be determined as

$$M = X + r*c_k + R*n_k$$

with r being the actual (but so far unknown) pupil radius, and correspondingly for the further eye, with primed quantities, at any given time $t_k \sim t'_k$, one arrives at the condition for the distance $\|M-M'\|$ between the eyeball centers

$$\|(X + r*c_k + R*n_k) - (X' + r'*c'_k + R'*n'_k)\| = IED \qquad (Eq.2)$$

in which $\|.\|$ denotes the length or norm of a vector. If one makes the physiologically plausible assumptions that R = R' (eyeballs of equal size, this is optional though) and r=r' (pupil radii are equal in both eyes at any given time), (Eq.2) can be rewritten

$$\|a + r*b\| = IED$$

where $a := X - X' + R*(n_k - n'_k)$ and $b := c_k - c'_k$. This leads to a quadratic equation for pupil radius r, which has the solutions

$$r_{1,2} = ( - a \cdot b \pm sqrt( (a \cdot b)^2 - \|b\|^2*(\|a\|^2 - IED^2) ) ) / \|b\|^2 \qquad (Eq.3)$$

with sqrt() signifying the square root operation and (a·b) signifying the dot product between these two vectors. The right side of (Eq.3) only contains known or measured quantities.

Which of the two solutions is the correct pupil radius can be easily decided either based on comparison with physiologically possible ranges (e.g. r > 0.5 mm and r < 4.5 mm) and/ or based on the geometric layout of the cameras and eyeballs. In Fig.3 for example, there would be a second solution at which eye intersecting lines $D_1$ and $D'_1$ comprise points M and M' at a distance of IED from each other, outside of the figure to the right, corresponding to the larger of one of the two values $r_{1,2}$, and for which the eyes would actually have swapped places. In this scenario, the smaller of values $r_{1,2}$ is therefore always the correct solution.

All the above calculations are performed with respect to a common 3D coordinate system, which can be the 3D coordinate system defined by a single camera of the device, or any other arbitrarily chosen coordinate system into which quantities have been transformed via the known relative camera poses, as is the case in the example of Fig.3.

Therefore, in this example algorithm a particularly simple and even faster solution for calculating all of the 3D eyeball centers, the optical axes (gaze vectors $g_k$, $g'_k$, which are antiparallel to $n_k$, $n'_k$ respectively) and the (joint) pupil size of both eyes is provided in a glint-free scenario based on merely a single observation in time of two eyes of a subject.

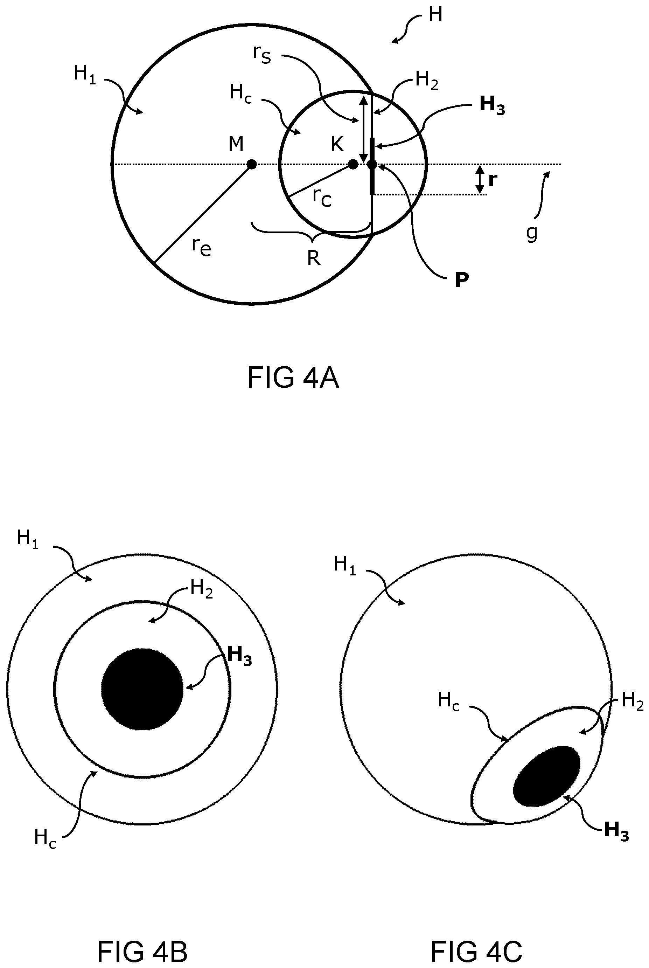

Reference will now be made to Figs. 4A to 6B, to illustrate embodiments of methods according to the invention.

As has been set out previously, given prior art algorithms for calculating eye state variables based on eye video/image data and 3D eye models often use very simple eye models, like for example the model with a single parameter R used in [1] or [4]. Such algorithms work on image data of real eyes, i.e. eyes which have a cornea, but utilize eye models which do not include such a cornea. Consequently, they can only determine approximations to the actual eye state variables and need strategies to correct them. This is achieved in the prior art by either employing computationally costly iterative numerical optimization methods, or by performing extensive simulations with synthetic data to provide multivariate polynomial post-hoc correction mappings or functions. According to the invention, a simpler method to generate data suitable for determining eye state variables, and an even faster and accurate method for determining such eye state variables based on existing algorithms that assume very simple 3D eye models are provided.

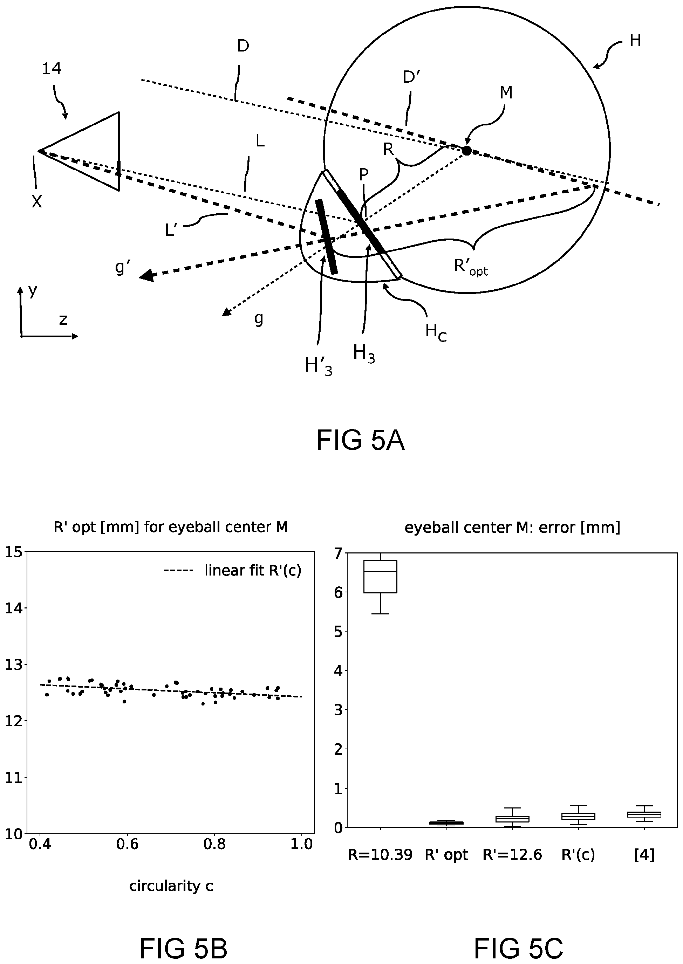

The underlying insights are illustrated with reference to Fig.5A. Fig.5A shows a cut through a 3D eye model similar to the ones of Figs. 2AB or Fig.3, symbolized by an eyeball H with its center M, pupil $H_3$ which is a circle in 3D with center P, and gaze vector g, which is the direction vector of the connection line between M and P, which at the same time is the normal vector to the iris-pupil plane. If an eye had no cornea, as detailed in connection with Figs. 2 to 3, example algorithms can derive eye state variables like the (pupil) circle center line L, the gaze vector, respectively optical axis, respectively pupil circle normal vector g, and - by utilizing a (in this case the single) parameter R of the 3D eye model - eye intersecting lines D.

A first insight of the invention is that, even though in real eyes a cornea $H_c$ distorts the apparent pupil (and hence the pupil image in the eye camera image) in a complex non-linear way, some aspects of this complex distortion can be summarized in a simple way. Namely, due to the refractive effects of the cornea, the apparent pupil $H'_3$ appears both further away from the eyeball center M as well as tilted towards the observing camera. Note that in Fig.5A a cornea $H_c$ is only depicted for the sake of illustrating the fact that in real eyes a modified, distorted apparent pupil $H'_3$ is perceived by an observer (like camera 14). The 3D eye model which in this example would be used by a given algorithm to derive eye state variables is one that has only one parameter (R) and does not model a cornea, just like the models depicted in Figs. 6AB.

If given prior art algorithms are applied to such a distorted pupil image, the resulting eye state variables, indicated in Fig.5A as a pupil circle center line L' and a gaze vector g' deviate from the actual variables. The same would be true for a corresponding eye intersecting line and eyeball center. It's a further insight of the invention however, that for any given combination of eye state variables, it is possible to find an adapted, hypothetically optimal value for a given parameter of the 3D eye model, which minimizes the error in determination of one or several selected eye state variables when used in the given algorithm in conjunction with the thus adapted 3D eye model. In the example of Fig.5A, it is possible to find an optimal value $R'_{opt}$, which in this case has to be used instead of the otherwise constant physiologically average value of R=10.39mm, in order to generate an eye intersecting line D' which actually comprises the eyeball center M. In this particular example, both high level pupil distortion effects mentioned, the apparent tilt towards the camera and the apparent distancing of the pupil from the eyeball center, combine to require $R'_{opt}$ to be larger than the physiologically average standard value of R.

Note that this insight is broadly applicable, in the sense that it is independent of the particular algorithm, the particular 3D eye model, the particular eye model parameter and the particular eye state variable. The algorithm used for determining eye state variables including the 3D eye model can in principle be a "black box" as long as the possibility is provided to inject different values for the parameter of the model which is to be optimized with respect to a certain eye state variable. The optimal value can be found via numeric optimization in a simulation scenario based on synthetic data in the following way.

A first 3D eye model modeling corneal refraction is chosen. As an example, a two-sphere eye model may be used to model eyeballs and corneal surfaces. For example, the so-called LeGrand eye model may be used, a schematic of which is presented in Fig.4A. It approximates the eye geometry as consisting of two partial spheres. The larger partial sphere $H_1$

corresponds to the eyeball with center at position M and radius of curvature $r_e$. The second partial sphere $H_C$ represents the cornea with center K and radius of curvature $r_c$. It is assumed that the cornea and the aqueous humor form a continuous medium with a single effective refractive index, $n_{ref}$. While the effective refractive index of the cornea varies slightly across the human population, it's average physiological value is assumed as $n_{ref}$ = 1.3375. The iris $H_2$ and pupil $H_3$ within the LeGrand eye model are two circles with radius $r_s$ and r, respectively, sharing the same center P lying at a distance R = $(r_e^2 - r_s^2)^{0.5}$ from M along the direction MK. Their normal directions coincide, are parallel to MK, and thus correspond to the optical axis g of the eye. The following physiological average values can be assumed: distance MP = R = 10.39 mm, eyeball radius of curvature re = 12 mm, cornea radius $r_c$ = 7.8 mm, and iris radius rs = 6 mm. The pupil radius r typically varies in the physiologically plausible range of approximately 0.5-4.5 mm.

Alternatively, the so-called Navarro eye model (see reference [2]) or any other 3D eye model which include a model of a cornea may be used for modeling eyes and generating synthetic images, respectively.

According to such a chosen, first 3D eye model which models corneal refraction, for a plurality of sets of chosen eye state variables defining different possible states of the 3D eye model, synthetic images of the thus obtained eyes can be generated using known (optical) camera properties (typically including camera intrinsics) of the camera intended to be used in a corresponding device for producing image data of a subject's eye.

Generating the synthetic images may be achieved by raytracing an arrangement of a camera model, which describes the camera, and 3D model eyeballs according to the first 3D eye model arranged in the field of view of the camera model.

The model of the camera typically includes a focal length, a shift of a central image pixel, a shear parameter, and/or one or more distortion parameters of the camera. The camera may be modeled as a pinhole camera. Typically, the camera defines a co-ordinate system, wherein all calculations described herein are performed with respect to this co-ordinate system.

These synthetic images are used to determine (calculate) expected values of the one or more eye state variables, using a given algorithm. Said given algorithm uses a further 3D eye model having at least one parameter. It is emphasized that the first 3D eye model, which is used to generate the synthetic images, is required to model corneal refraction, while the further 3D eye model, used by the given algorithm to determine eye state variables, can be a simpler model, in particular one that does not comprise a cornea, in particular even an eye model with just a single parameter.

The chosen eye state variable values typically include co-ordinates of respective centers of the model eyeballs, given radii of a pupil of the model eyeballs and/or given gaze directions of the model eyeballs. Two examples of such images are presented in Fig.4B and Fig.4C.

Given one or several of such synthetic images, the given algorithm calculates one or more eye state variables, and a numeric optimization determines the hypothetically optimal value or values of one or more parameters of the further 3D eye model (used by the algorithm) which minimize(s) the error between the (calculated) expected value of one or more eye state variables and the corresponding chosen (ground truth) values. The algorithm might take a single synthetic image as input to calculate a certain eye state variable, and thus a hypothetically optimal value of the one or more parameters may be obtained for each synthetic image, or the algorithm might operate on an ensemble of several synthetic images.

Referring again to the example of Fig.5A, in this particular case the optimal value $R'_{opt}$ that needs to be used instead of the distance R=PM between the pupil center and the eyeball center, the only parameter in this particular further 3D eye model, is determined such that the eye state variable D', the eye intersecting line, correctly runs through, or at least as close as possible to the true eyeball center M. In this example, since one eye intersecting line can be obtained from a single image, an optimal value $R'_{opt}$ can be obtained for each synthetic image generated.

It shall be emphasized at this point, that said numerical optimization is fundamentally different from optimization based methods of the prior art which have been previously referenced. Prior art methods use iterative numerical optimization schemes to derive the eye state variables themselves, based on time-series of real eye image data. Therein lies their weakness, since they cannot operate in real-time due to the computational complexity and the high frame rates encountered in state of the art systems for eye state variable determination. In contrast thereto, the methods presented herein provide means to adapt simple eye models based on simulation data which can be pre-computed in a non time critical manner. In other words, according to the invention a method for generating data suitable for determining eye state variables may use iterative numerical optimization techniques in order to generate such data, because at that stage calculations are not time critical, thereby enabling the use of non-iterative algorithms in methods for determining said eye state variables, where speed of calculation is of utmost importance.

The hypothetically optimal value(s) of one or more parameters of the further 3D eye model constitute data suitable for determining at least one eye state variable of at least one eye of a subject, and their application and use therefore will be detailed in the following example embodiments.

As a further insight, the inventors have surprisingly found that it is possible to find generalizable relationships between said optimal values and characteristics of the (camera) image of the pupil. Embodiments thus include establishing a relationship between the hypothetically optimal value(s) of the at least one parameter of the further 3D eye model and a characteristic of the pupil image.

According to a preferred embodiment, the characteristic of the image of the pupil is a measure of the circularity (c) of the pupil area or outline, in particular a ratio of minor to major axis length of an ellipse fit to the pupil image area, a measure of variation of the curvature of the pupil outline, a measure of elongation of the pupil or a measure of the bounding box of the pupil area.

Despite the complex dependency of the shape of the pupil image on the pose and pupil radius of the eye due to corneal refraction, it has surprisingly been found that a measure of the shape which represents the pupil in the camera image, like for example a circularity measure, which can be very easily obtained from given image data in real-time, makes it possible to find simple relationships which make the parameter(s) of the 3D eye models of the prior art adaptive to account for the effects of corneal refraction in a very simple and efficient way.

Reference is made to Figs. 5B to 5C for an example. In Fig.5B, optimal values of the (single) eye model parameter $R'_{opt}$ as discussed in connection with Fig.5A have been plotted (dots) for a small number of synthetic eye images generated using different eye state variables. As can be seen, only a very small number of synthetic observations suffices to demonstrate a relationship between the optimal values of the parameter of the further 3D eye model used and pupil image circularity, when using a given algorithm to determine an eye state variable (in this case the eye intersecting line D', based on which the eyeball center M can be determined).

In the context of the disclosure, a relationship between the hypothetically optimal values of the at least one further 3D eye model parameter and the characteristic of the pupil image is to signify any numerical link between these two quantities, for example also a constant value. Such constant value can for example be an average value of the optimal eye model parameter over a certain range of pupil characteristic values.

In particular a constant value smaller or larger than the corresponding average parameter of the first 3D eye model can be used.

In the example of Fig.5B, an average value of $<R'_{opt}>$ = 12.6 mm could for example be derived as the relationship with the pupil characteristic of circularity, which in this particular case of determining the eye intersecting line as the eye state variable, is a value larger than the physiologically average human value of R = 10.39 mm used in the first 3D eye model, as illustrated in Fig.5A.

Other relationships include a linear relationship, such as a linear least-squares fit, as indicated by the dashed line in Fig.5B, a polynomial fit, and a general non-linear fit.

Fig.5C shows residual errors produced by a given algorithm determining the eyeball center as an eye state variable, when using different values of the eye model parameter being the distance between pupil center and eyeball center, in a simulation with synthetic images. Note again that, unlike the first 3D eye model which models corneal refraction and which is used for generating the synthetic image data on which the algorithm then operates, the 3D eye model used by the algorithm to determine eye state variables is ignorant per se of any cornea and in this example just models the eye sphere size via distance R as a single model parameter. In the leftmost column of Fig.5C, it can be seen that assuming the average physiological human value R = 10.39 mm produces an average error in 3D eyeball position determination of between 6-7mm. Using the optimal value for every observation (synthetic eye image), see second column, the error is indeed minimized. It is not exactly zero due to numerical discretization errors and the simplifying assumptions underlying the schematic of Fig.5A. The third and fourth column show the residual error when using either a constant value respectively the linear fit as indicated in Fig.5B. Finally, the last, fifth column shows the residual error when applying the post-hoc refraction correction function/scheme as described in [4] to the data of the first column of Fig.5C, i.e. to the eye state variable values as obtained with a 3D eye model which uses the non-adapted parameter R = 10.39 mm.

As can be seen from Fig.5C, both using a (optimal) constant or a (optimal) linear relationship between the 3D eye model's parameter R' and the pupil characteristic c reduces the error in determining the eye state variable equally well, and both are more accurate than the prior art methods.

While the number of eye image frames that can be processed with a method such as described in [4] is, for the same computing hardware, already typically at least one order of magnitude higher compared to the method of Swirski (reference [1]), application of such post-hoc correction mapping of the prior art typically can take of the order of 10-100 microseconds per eye image/observation, depending on the complexity of the mapping, like the polynomial degree. In contrast, the method of the present invention requires either zero additional calculation time at runtime if a constant (optimal) value for the adapted parameter of the further 3D eye model is used (column three of Fig.5C), or requires only calculation of the pupil shape characteristic plus either a simple look-up operation or very few floating point operations for calculating the optimal parameter given the relationship, which can for example be linear (column 4 of Fig.5C). The latter can typically be obtained with operations taking of the order of 100 nanoseconds, i.e. a factor 1000 faster than even the prior art methods like [4].

A further advantage of the methods of the present invention is that they are entirely independent of the choice of any coordinate system, unlike prior art methods like [4] which apply a multi-dimensional correction mapping to a set of eye state variables which may only be defined at least partly in a particular coordinate system (e.g. eyeball center coordinates, eye intersecting line directions, gaze vector directions, etc.). In contrast, the methods of the present invention operate by adapting parameters of the (further) 3D eye model, which are entirely independent of any choice of particular coordinate system that the algorithm for determining eye state variables might be using.

According to other embodiments, the further 3D eye model may have more than one parameter and a relationship may be established for more than one of them.

According to embodiments, the relationship may be the same for all eye state variables, or a different relationship between a (any) parameter of the (further) 3D eye model and the characteristic of the pupil image may be established for each eye state variable or for groups of eye state variables.

For example, eye state variables may be selected from the non-exhaustive list of a pose of an eye such as a location of an eye, in particular an eyeball center, an orientation of an eye, in particular a gaze vector, optical axis orientation or visual axis orientation, a 3D circle center line, a 3D eye intersecting line, and a size measure of a pupil of an eye, such as a pupil radius or diameter.

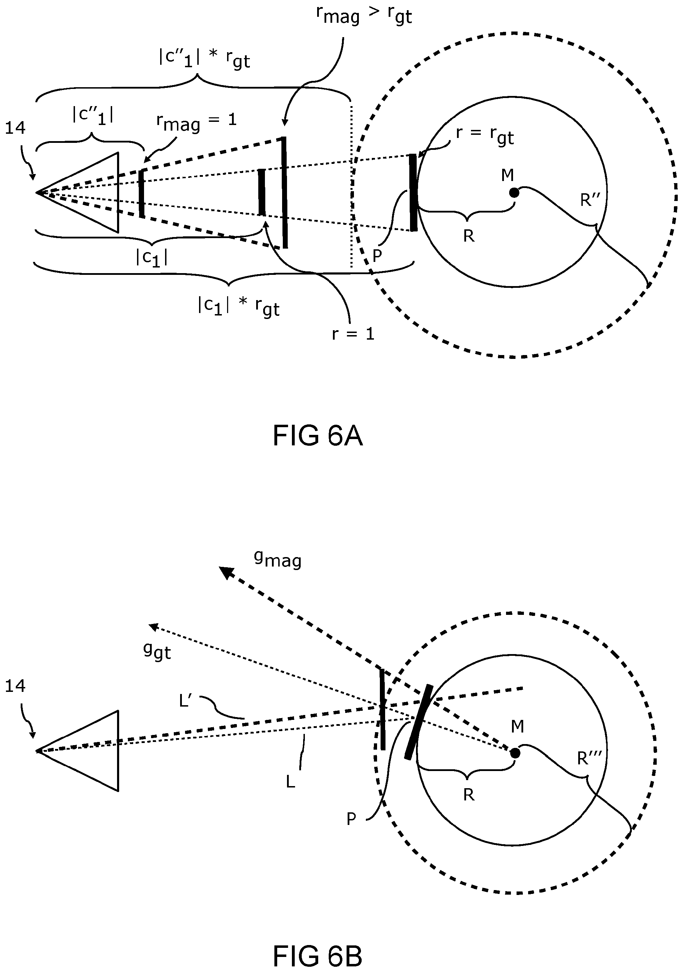

Figs. 6A and 6B provide examples of further eye state variables for which an individual optimal relationship between a parameter of the further 3D eye model and a pupil image characteristic may be established.

Referring to Fig.6A, an example of how a parameter of a further 3D eye model may be adapted for taking into account effects of corneal refraction during determination of the eye state variable pupil size is presented. Once the center of the eyeball M has been determined, one possible method to determine the actual radius of the pupil $r_{gt}$ proceeds as follows.

As has been previously detailed in connection with the monocular and binocular algorithms and the mathematical methods therefore referenced in [1] and [3], having detected the elliptical shape best approximating the pupil in a camera image of an eye, a set of parallel shifted circles in 3D can be calculated, said circles increasing in radius as the distance from the camera (center of perspective projection) increases, their centers forming a circle center line. As long as the location of the eye is unknown, said size-distance ambiguity exists. Once the center of the eye M is known, the circle which lies tangent to an eye sphere of radius R, where R=PM represent the assumed distance between the pupil center P and the eyeball center M, represents the actual pupil circle in 3D. Its radius can for example be determined by first finding the circle of radius r = 1 mm along the circle center line. The center of this circle is designated by its vector $c_1$ as previously explained, e.g. with (Eq.1). Shifting, that is scaling this circle such that it lies tangent to the eye sphere of center M and radius R will bring the center of the circle to a distance $|c_1| * r_{gt}$ from the camera center, and the radius $r_{gt}$ of the pupil has thus been found. This is illustrated schematically (not to scale) in Fig.6A.

This procedure is however only correct for an eye which has NO cornea. Corneal refraction adds effects of non-linear distortion to the image of the pupil. In particular, the cornea "magnifies" the apparent pupil. This is synonymous to saying that the eye constitutes a fish-eye camera/lens - the cornea allows it to collect light from a wider angle than it would be able without a cornea. This magnification has been symbolized in Fig.6A by an apparent pupil which appears bigger than the actual pupil or closer to the camera (both effects have been shown in Fig.6A to make the effect clearer and NO cornea has been drawn for the sake of clarity).

The unprojection cone of the magnified pupil of apparent radius $r_{mag} > r_{gt}$, which has been indicated in Fig.6A by fat dashed lines, thus has a larger opening angle than the one of the actual pupil, indicated by finer dashed lines. Hence, the circle with radius $r_{mag} = 1$ mm which lies closer to the camera, at a distance $|c''_1|$. Since $|c''_1| < |c_1|$, scaling this circle until it lies tangent to an eye sphere of center M and radius R would yield a pupil radius which would be too large. Hence, according to another example of the invention, a hypothetically optimal value for the parameter of the further 3D eye model which represents the distance between eyeball center and pupil center can be determined for any eye observation in a simulation scenario as previously detailed. In Fig.6A this is indicated by an optimal value R".

Referring to Fig.6B, another example of how a parameter of a further 3D eye model may be adapted for taking into account effects of corneal refraction during determination of an eye state variable is presented, the eye state variable being the gaze vector in this example.

One possible way of determining a gaze vector is to directly use the circle normal vector, as provided by the "unprojection" of the pupil image ellipse (based on methodology described in reference [3]), see vectors g respectively g' in Fig.5A. This strategy can however yield a gaze vector which is subject to substantial noise. Therefore, once the center of the eyeball M has been determined, one possible alternative method to determine the actual orientation, optical axis or gaze direction of the eye proceeds as follows.

Having detected the elliptical shape best approximating the pupil in a camera image of an eye and the corresponding pupil circle center line L as detailed herein already in connection with Figs. 2ABC, 3 and 5A, once the eyeball center M is known, one possible way of determining the direction vector g of the optical axis of the eye is to intersect said circle center line L with the eye sphere of center M and radius R, which yields the pupil center P. The normal vector to the sphere surface in the pupil center point P is the desired vector g.

Again, this procedure is however only correct for an eye which has NO cornea. As has been explained in connection with Fig.5A, the apparent image of the pupil appears further away from the eyeball center M and tilted towards the camera, thus giving rise to a tilted circle center line L'. Applying the strategy outlined, a wrong gaze vector $g_{mag}$ thus results, as indicated in Fig.6B (again, a cornea has been omitted for the sake of clarity).

However, it has been found that also in this example of the determination of another eye state variable, a hypothetically optimal value for a parameter of the further 3D eye model, in this case the distance which represents the distance between eyeball center and pupil center, can be determined for any eye observation in a simulation scenario as previously detailed. In Fig.6B this is indicated by an optimal value R‴. Finding a relationship between said optimal value and a pupil image characteristic, as before, enables an algorithm for determining eye state variables, which employs a simple further 3D eye model that is per se agnostic about corneal refraction, to leverage the advantages of the methods of the invention.

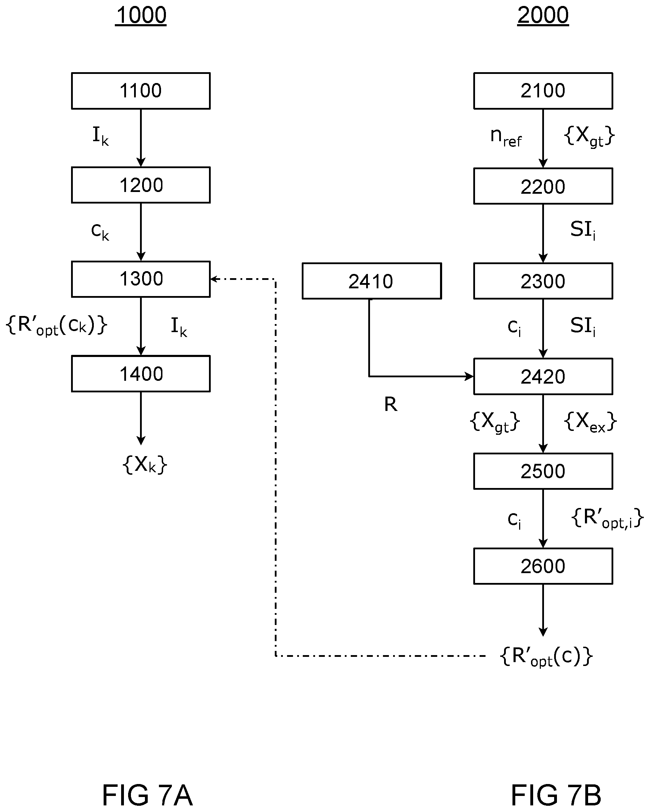

Referring now to Figs. 7A and 7B, flow charts of methods according to embodiments will be explained.

Fig.7B illustrates a flow chart of a method 2000 for generating data suitable for determining at least one eye state variable of at least one eye of a subject according to embodiments.

In a first step 2100, a first 3D eye model modeling corneal refraction is provided.

In a second step 2200, synthetic images SIi of several model eyes H with corneal refractive properties symbolized by an effective corneal refraction index $n_{ref}$ in the flow chart and a plurality of given values {Xgt} of one or more eye state variables {X} of the model eye are generated using a model of the camera such as a pinhole model, assuming full perspective projection. For example, a ray tracer may be used to generate the synthetic images. For accuracy reasons, synthetic images may be ray traced at arbitrarily large image resolutions.

Eye state variables may for example include eyeball center locations M, gaze vectors g and pupil radii r, and may be sampled from physiologically plausible ranges as well as value ranges that may be expected for a given scenario, such as head-mounted eye cameras or remote eye tracking devices. For example, after fixing $M_{gt}$ at a position randomly drawn from a range of practically relevant eyeball positions corresponding to a typical geometric setup of the eye camera, a number of synthetic eye images are generated, with gaze angles $\varphi$ and $\theta$ (forming $g_{gt}$) randomly chosen from a uniform distribution between physiologically plausible maximum gaze angles, and with pupil radii $r_{gt}$ randomly chosen from a uniform distribution between 0.5mm and 4.5mm. Typically, a small number N of eye state variable tuples {gex, rex, Mex}i, {$g_{gt}$, $r_{gt}$, $M_{gt}$}i with i=1...N suffices to establish a relationship between the hypothetically optimal values of the further 3D eye model parameter and the pupil characteristic in a later step. For example, N maybe of the order of $10^3$ or even only $10^2$.

Eye model parameters may or may not be subject to variation in this step. In particular, they may be set to constant physiologically average values as for example detailed in connection with the eye model of Fig.4A. They may also be drawn from known physiological statistical distributions.

In step 2300, a characteristic $c_i$ of the image of the pupil within each of the synthetic images $SI_i$ is determined.

The characteristic may for example be a measure of the circularity of the pupil area or outline, in particular a ratio of minor to major axis length of an ellipse fit to the pupil image area, a measure of variation of the curvature of the pupil outline, a measure of elongation of the pupil or a measure of the bounding box of the pupil area.

In step 2410, a further 3D eye model having at least one parameter R is provided.

In particular, the further 3D eye model can be different from the first 3D eye model, in particular simpler. The further 3D eye model can have multiple parameters, but can in particular also have a single parameter R, which for the sake of clarity is the case illustrated in this flow chart.

In step 2420, a given algorithm is used to calculate one or more eye state variables {$X_{ex}$} using one or more of the synthetic images $SI_i$ and the further 3D eye model having at least one parameter R. As explained previously in more detail with regard to a monocular and a binocular algorithm for determining eye state variables, the expected values of the one or more eye state variables {$X_{ex}$} can be determined according to any suitable algorithm.

Thereafter, in step 2500, the given values {Xgt} and the calculated, expected values {$X_{ex}$} of one or more eye state variables {X} are used in an error minimization step to determine one or more hypothetically optimal values of the at least one parameter of the further 3D eye model that minimize the error between the values of the corresponding at least one given eye state variable and the value of the (calculated respectively expected) eye state variable obtained when applying the given algorithm.

The superscript ' in R' indicates that the value of the parameter R is being changed from its original value, and the subscript opt in $R_{opt}$ indicates that it is optimal in some sense. The curly brackets {.} indicate, that the parameter may be optimized for calculating a (each) particular eye state variable or group of eye state variables, such that a set of relationships of optimal parameters {$R'_{opt}(c)$} results. Alternatively, only one such relationship may be determined for a certain parameter, which relationship can then be used by a given algorithm to calculate all possible eye state variables.

Finally, in step 2600 a relationship between the hypothetically optimal values of the at least one parameter of the further 3D eye model and the characteristic of the pupil image is established. The relationship(s) may be stored in a memory (not shown).

Steps of the method as detailed with reference to Fig.7B may be performed by a computing and control unit of a system, such as a personal computer, laptop, server or cloud computing system, thereby forming a system for generating data suitable for determining at least one eye state variable of at least one eye of a subject, according to embodiments.

Fig.7A illustrates a flow chart of a method 1000 for determining at least one eye state variable of at least one eye of a subject according to embodiments.

In a first step 1100, image data $I_k$ of the user's eye, taken by an eye camera of known camera intrinsics of a device at one or more times $t_k$ is received.

Said image data may consist for example of one or several images, showing one or several eyes of the subject.

In a subsequent step 1200, a characteristic of the image of the pupil within the image data is determined. In case said image data comprises multiple images, such characteristic is determined in each image, and if the image data comprises images of multiple eyes, such characteristic may be determined for each eye separately.

In a subsequent step 1300, a 3D eye model having at least one parameter R is provided, wherein the parameter depends in a pre-determined relationship on the characteristic.

In step 1400, a given algorithm is used to calculate the at least one eye state variable {X} using the image data $I_k$ and the 3D eye model including the at least one characteristic-dependent parameter.

The given algorithms used in steps 2420 and 1400 may for example employ methods such as the monocular or binocular algorithms previously explained with regard to Figs. 2ABC and 3.

The further 3D eye model provided in step 2410 and the 3D eye model provided in step 1300 may be the same or different ones, as long as they comprise a corresponding parameter or corresponding parameters {R} for which optimal relationships in the sense of step 2600 have been determined.

According to the present disclosure, methods for generating data suitable for determining eye state variables are provided, which open the way to a fast non-iterative approach to the tasks of refraction-aware 3D gaze prediction and pupillometry based on pupil contours alone. Leveraging geometrical insights with regard to the two-sphere eye model and/or with regard to human ocular physiology, in particular the distortion of the image of the pupil due to corneal refraction, these tasks are solved by making simple 3D eye models adaptive, which virtually eliminates the systematic errors due to corneal refraction of prior art methods.

## References

[1] Swirski L. et al: A fully-automatic, temporal approach to single camera, glint-free 3D eye model fitting, Proc. PETMEI Lund/Sweden, 13.08.2013

[2] Navarro R. et al: Accommodation-dependent model of the human eye with aspherics, J. Opt. Soc. Am. A 2(8), 1273-1281 (1985)

[3] Safaee-Rad R. et al: Three-dimensional location estimation of circular features for machine vision, IEEE Transactions on Robotics and Automation 8(5), 624-640 (1992)

[4] Dierkes K. et al: A fast approach to refraction-aware eye-model fitting and gaze prediction, Proc. ETRA Denver/USA, 25.-28.06.2019

[5] Fedtke C. et al: The entrance pupil of the human eye: a three-dimensional model as a function of viewing angle. Optics Express 18(21), 22364-22376 (2010)

## Reference numbers

**[0210]**

1 head wearable device, head wearable spectacles device
2 main body, spectacles body
3 nose bridge portion
4 frame
5 illumination means

7 computing and control unit

10 left side
11 left ocular opening
12 left lateral portion
13 left holder / left temple (arm)
14 left camera
15 optical axis (left camera)

17 left inner eye camera placement zone
18 left outer eye camera placement zone
19 left eye

| 20 | right side |
|---|---|
| 21 | right ocular opening |
| 22 | right lateral portion |
| 23 | right holder / right temple (arm) |
| 24 | right camera |
| 25 | optical axis (right camera) |

| 27 | right inner eye camera placement zone |
|---|---|
| 28 | left outer eye camera placement zone |
| 29 | right eye |
| 30 | bounding cuboid |
| 31 | left lateral surface |
| 32 | right lateral surface |
| 33 | upper surface |
| 34 | lower surface |

| 100 | middle plane |
|---|---|
| 101 | horizontal direction |
| 102 | vertical direction |
| 103 | down |
| 104 | up |
| 105 | front |
| 106 | back |

| $\alpha,\gamma$ | angle of inner/outer left camera 14 |
|---|---|
| $\beta,\delta$ | angle of inner/outer right camera 24 |

| >= 1000 | methods, method steps |
|---|---|

## Claims

1. A computer-implemented method for generating data suitable for determining at least one eye state variable of at least one eye of a subject, the eye comprising an eyeball, an iris defining a pupil, and a cornea, the at least one eye state variable being derivable from at least one image of the eye taken with a camera of known camera intrinsics, the method comprising:

   - providing a first 3D eye model modeling corneal refraction;
   - generating, using the known camera intrinsics, synthetic images of several model eyes according to the first 3D eye model, for a plurality of given values of at least one eye state variable;
   - using a given algorithm to calculate the at least one eye state variable using one or more of the synthetic images and a further 3D eye model having at least one parameter;
   - determining a characteristic of a pupil image within each of the synthetic images;
   - determining one or more hypothetically optimal values of the at least one parameter of the further 3D eye model that minimize the error between the value(s) of the at least one given eye state variable and the value(s) of the corresponding eye state variable obtained when applying the given algorithm; and
   - establishing a relationship between the one or more hypothetically optimal values of the at least one parameter of the further 3D eye model and the characteristic of the pupil image.

2. The method of claim 1, wherein the characteristic of the pupil image is a measure of the circularity of the pupil area or outline, in particular a ratio of minor to major axis length of an ellipse fit to the pupil image area or outline, a measure of variation of the curvature of the pupil outline, a measure of elongation or a measure of the bounding box of the pupil area, and/or wherein the relationship between the hypothetically optimal values of the at least one further 3D eye model parameter and the characteristic of the pupil image is chosen from the list of a constant value, in particular a constant value smaller or larger than the corresponding average parameter of the first 3D eye model, a linear relationship, a polynomial relationship, or another non-linear relationship, in particular a relationship derived via a regression fit.

3. The method of any preceding claim, wherein the further 3D eye model has at most one parameter, or wherein the further 3D eye model has multiple parameters and a relationship is established for more than one of them.

4. The method of any preceding claim, wherein any parameter of the first and/or of the further 3D eye model is/are selected from the list of a distance between a center of an eyeball, in particular a rotational, geometrical or optical center, and a center of a pupil or cornea, a size measure of an eyeball, a cornea or an iris such as an eyeball radius, a cornea radius, an iris diameter, a distance pupil center to cornea center, a distance cornea center to eyeball center, a distance pupil center to limbus center, a distance crystalline lens to eyeball center, to cornea center and/or to corneal apex, a refractive property of an eye structure such as an index of refraction of a cornea, vitreous humor or crystalline lens, an ellipsoidal shape measure of an eyeball or cornea, and a degree of astigmatism.

5. The method of any preceding claim, wherein said relationship is the same for all eye state variables, or wherein a different relationship between a parameter of the further 3D eye model and the characteristic of the pupil image is established for each eye state variable or for groups of eye state variables.

6. The method of any preceding claim, wherein the eye state variable is selected from the list of a pose of an eye such as a location of an eye, in particular an eyeball center, and/or an orientation of an eye, in particular a gaze vector, optical axis orientation or visual axis orientation, a 3D circle center line, a 3D eye intersecting line, and a size measure of a pupil of an eye, such as a pupil radius or diameter.

7. A computer-implemented method for determining at least one eye state variable of at least one eye of a subject, the eye comprising an eyeball, an iris defining a pupil, and a cornea, the at least one eye state variable being derivable from at least one image of the eye taken with a camera of known camera intrinsics, the method comprising:

   - receiving image data of the at least one eye from a camera of known camera intrinsics and defining an image plane;
   - determining a characteristic of a pupil image within the image data;
   - providing a 3D eye model having at least one parameter, the at least one parameter depending in a pre-determined relationship on the characteristic; and
   - using a given algorithm to calculate the at least one eye state variable using the image data and the 3D eye model including the at least one characteristic-dependent parameter.

8. The method of claim 7, wherein the characteristic of the pupil image is a measure of the circularity of the pupil area or outline, in particular a ratio of minor to major axis length of an ellipse fit to the pupil image area or outline, a measure of variation of the curvature of the pupil outline, a measure of elongation or a measure of the bounding box of the pupil area, and/or wherein the pre-determined relationship between the at least one parameter of the 3D eye model and the characteristic of the pupil image is chosen from the list of a constant value, a linear relationship, a polynomial relationship, or another non-linear relationship, in particular a relationship derived via a regression fit, in particular wherein the relationship is stored in analytical form and evaluated on-the-fly for given image data or stored as a lookup-table, LUT.

9. The method of any of claims 7 to 8, wherein the 3D eye model has at most one parameter or has multiple parameters and a pre-determined relationship between any of them and the characteristic is used for at least one of the parameters.

10. The method of any of claims 7 to 9, wherein any parameter of the 3D eye model is selected from the list of a distance between a center of an eyeball, in particular a rotational, geometrical or optical center, and a center of a pupil or cornea, a size measure of an eyeball, a cornea or an iris such as an eyeball radius, a cornea radius, an iris diameter, a distance pupil-center to cornea-center, a distance cornea-center to eyeball-center, a distance pupil-center to limbus center, a distance crystalline lens to eyeball-center, to cornea center and/or to corneal apex, a refractive property of an eye structure such as an index of refraction of a cornea, vitreous humor or crystalline lens, an ellipsoidal shape measure of an eyeball or cornea, and a degree of astigmatism, wherein said relationship is the same for all eye state variables, or wherein a different pre-determined relationship between a parameter of the 3D eye model and the characteristic of the pupil image is used for each eye state variable or for groups of eye state variables, and/or wherein any such relationship has been established according to the method of any of claims 1 to 6, and/or wherein the eye state variable is selected from the list of a pose of an eye such as a location of an eye, in particular an eyeball center, and/or an orientation of an eye, in particular a gaze vector, optical axis orientation or visual axis orientation, a 3D circle center line, a 3D eye intersecting line, and a size measure of a pupil of an eye, such as a pupil radius or diameter.

11. The method of any preceding claim, wherein the given algorithm does not take into account a glint from the eye for calculating the at least one eye state variable, and/or wherein the algorithm is glint-free, and/or wherein the algorithm does not require structured light and/or special purpose illumination to derive eye state variables, wherein the given algorithm calculates the at least one eye state variable in a non-iterative way, and/or wherein the given algorithm includes:

- determining a first ellipse in the image data, the first ellipse at least substantially representing a border of the pupil of the at least one eye at a first time ($t_1$);
- using the camera intrinsics and the first ellipse to determine a 3D orientation vector of a first circle in 3D and a first center line on which a center of the first circle is located in 3D, so that a projection of the first circle, in a direction parallel to the first center line, onto the image plane is expected to reproduce the first ellipse; and
- determining a first eye intersecting line in 3D expected to intersect a 3D center of the eyeball at the corresponding time ($t_1$) as a line which is, in the direction of the orientation vector, parallel-shifted to the first center line by an expected distance between the center of the eyeball and a center of the pupil.

12. The method of claim 11, further comprising:

- receiving image data of a further eye of the subject at a time ($t'_1$), substantially corresponding to the first times ($t_1$), from a camera of known camera intrinsics and defining an image plane, the further eye comprising a further eyeball, a further iris defining a further pupil, and a further cornea, the given algorithm further including:

  - determining a further ellipse in the image data, the further ellipse at least substantially representing the border of the further pupil of the further eye at the corresponding time ($t'_1$);
  - using the camera intrinsics and the further ellipse to determine a 3D orientation vector of a further circle in 3D and a further center line on which a center of the further circle is located in 3D, so that a projection of the further circle, in a direction parallel to the further center line, onto the image plane is expected to reproduce the further ellipse; and
  - determine a further eye intersecting line in 3D expected to intersect a 3D center of the further eyeball at the corresponding time ($t'_1$) as a line which is, in the direction of the 3D orientation vector of the further circle, parallel-shifted to the further center line by an expected distance between the center of the further eyeball and a center of the further pupil.

13. A system for generating data suitable for determining at least one eye state variable of at least one eye of a subject, the eye comprising an eyeball, an iris defining a pupil, and a cornea, the at least one eye state variable being derivable from at least one image of the eye taken with a camera of known camera intrinsics, the system comprising a computing and control unit configured to:

- generate, using the known camera intrinsics, synthetic images of several model eyes according to a first 3D eye model modeling corneal refraction, for a plurality of given values of at least one eye state variable;
- calculate, using a given algorithm, the at least one eye state variable making use of one or more of the synthetic images and a further 3D eye model having at least one parameter;
- determine a characteristic of a pupil image within each of the synthetic images;
- determine one or more hypothetically optimal values of the at least one parameter of the further 3D eye model that minimize the error between the value(s) of the at least one given eye state variable and the value(s) of the corresponding eye state variable obtained when applying the given algorithm; and
- establish a relationship between the one or more hypothetically optimal values of the at least one parameter of the further 3D eye model and the characteristic of the pupil image and store it in a memory; and/or

wherein the computing and control unit is configured to perform the method of any of the claims 1 to 6.

14. A system for determining at least one eye state variable of at least one eye of a subject, the eye comprising an eyeball, an iris defining a pupil, and a cornea, the at least one eye state variable being derivable from at least one image of the eye taken with a camera of known camera intrinsics, the system comprising:

a device comprising at least one camera of known camera intrinsics for producing image data including at least one eye of a subject, the at least one camera comprising a sensor defining an image plane ($I_p$), the at least one eye comprising an eyeball, an iris defining a pupil, and a cornea; and
a computing and control unit configured to:

- receive image data of the at least one eye from the at least one camera;
- determine a characteristic of a pupil image within the image data;
- calculate, using a given algorithm, the at least one eye state variable making use of the image data and a 3D eye model having at least one parameter, the at least one parameter depending in a pre-determined relationship on the characteristic, the relationship being retrieved from a memory; and/or

wherein the computing and control unit is configured to perform the method of any of the claims 7 to 12.

15. A computer program product and/or a computer-readable storage medium comprising instructions which, when executed by a one or more processors of a system, in particular the system according to claim 13 or claim 14, cause the system to carry out the steps of the method according to any one of the claims 1 to 12.

**Patentansprüche**

1. Computerimplementiertes Verfahren zum Erzeugen von Daten, die geeignet sind, mindestens eine Augenzustandsvariable von mindestens einem Auge eines Subjekts zu bestimmen, wobei das Auge einen Augapfel, eine eine Pupille definierende Iris und eine Hornhaut aufweist, wobei die mindestens eine Augenzustandsvariable aus mindestens einem Bild des Auges ableitbar ist, das mit einer Kamera mit bekannten Kameraeigenschaften aufgenommen wurde, wobei das Verfahren umfasst:

   - Bereitstellen eines ersten 3D-Augenmodells, das eine Hornhautrefraktion modelliert;
   - Erzeugen, unter Verwendung der bekannten Kameraeigenschaften, von synthetischen Bildern mehrerer Modellaugen gemäß dem ersten 3D-Augenmodell für eine Vielzahl von gegebenen Werten mindestens einer Augenzustandsvariablen;
   - Verwendung eines gegebenen Algorithmus zur Berechnung der mindestens einen Augenzustandsvariablen unter Verwendung eines oder mehrerer der synthetischen Bilder und eines weiteren 3D-Augenmodells mit mindestens einem Parameter;
   - Bestimmung einer Eigenschaft eines Pupillenbildes in jedem der synthetischen Bilder;
   - Bestimmen eines oder mehrerer hypothetisch optimaler Werte des mindestens einen Parameters des weiteren 3D-Augenmodells, die den Fehler zwischen dem (den) Wert(en) der mindestens einen gegebenen Augenzustandsvariablen und dem (den) Wert(en) der entsprechenden Augenzustandsvariablen, die bei Anwendung des gegebenen Algorithmus erhalten werden, minimieren; und
   - Herstellen einer Beziehung zwischen dem einen oder mehreren hypothetisch optimalen Werten des mindestens einen Parameters des weiteren 3D-Augenmodells und der Eigenschaft des Pupillenbildes.

2. Verfahren nach Anspruch 1, wobei die Eigenschaft des Pupillenbildes ein Maß für die Kreisförmigkeit der Pupillenfläche oder des Pupillenumrisses ist, insbesondere ein Verhältnis von Neben- zu Hauptachsenlänge einer an die Pupillenbildfläche oder den Pupillenumriss angepassten Ellipse, ein Maß für die Variation der Krümmung des Pupillenumrisses, ein Maß für die Verlängerung oder ein Maß für die Bounding Box der Pupillenfläche, und/oder wobei die Beziehung zwischen den hypothetisch optimalen Werten des mindestens einen weiteren 3D-Augenmodell-Parameters und der Eigenschaft des Pupillenbildes aus der Liste eines konstanten Wertes, insbesondere eines konstanten Wertes, der kleiner oder größer ist als der entsprechende durchschnittliche Parameter des ersten 3D-Augenmodells, einer linearen Beziehung, einer polynomialen Beziehung oder einer anderen nichtlinearen Beziehung, insbesondere einer über eine Regressionsanpassung abgeleiteten Beziehung, ausgewählt wird.

3. Verfahren nach einem der vorhergehenden Ansprüche, wobei das weitere 3D-Augenmodell höchstens einen Parameter aufweist, oder wobei das weitere 3D-Augenmodell mehrere Parameter aufweist und für mehr als einen von ihnen eine Beziehung hergestellt wird.

4. Verfahren nach einem der vorhergehenden Ansprüche, wobei ein beliebiger Parameter des ersten und/oder des weiteren 3D-Augenmodells ausgewählt ist aus der Liste eines Abstands zwischen einem Mittelpunkt des Augapfels, insbesondere einem rotatorischen, geometrischen oder optischen Mittelpunkt, und einem Mittelpunkt einer Pupille oder Hornhaut, eines Größenmaßes eines Augapfels, einer Hornhaut oder einer Iris, wie eines Augapfelradius, eines Hornhautradius, eines Irisdurchmessers, ein Abstand Mittelpunkt der Pupille zum Mittelpunkt der Hornhaut, ein Abstand Mittelpunkt der Hornhaut zum Mittelpunkt des Augapfels, ein Abstand Mittelpunkt der Pupille zum Mittelpunkt des Limbus, ein Abstand der Augenlinse zum Mittelpunkt des Augapfels, zum Mittelpunkt der Hornhaut und/oder zum Scheitelpunkt der Hornhaut, eine Brechungseigenschaft einer Augenstruktur wie ein Brechungsindex der Hornhaut, des Glaskörpers oder der Augenlinse, ein Maß für die Ellipsoidform eines Augapfels oder der Hornhaut und ein Grad des Astigmatismus.

5. Verfahren nach einem der vorhergehenden Ansprüche, wobei die Beziehung für alle Augenzustandsvariablen gleich ist, oder wobei für jede Augenzustandsvariable oder für Gruppen von Augenzustandsvariablen eine unterschiedliche Beziehung zwischen einem Parameter des weiteren 3D-Augenmodells und der Eigenschaft des Pupillenbildes hergestellt wird.

6. Verfahren nach einem der vorhergehenden Ansprüche, wobei die Augenzustandsvariable aus der Liste einer Pose eines Auges, wie einer Position eines Auges, insbesondere eines Augapfels, und/oder einer Orientierung eines Auges, insbesondere eines Blickvektors, einer optischen Achsenausrichtung oder einer Blickachsenausrichtung, einer 3D-Kreis-Mittellinie, einer 3D-Augenschnittlinie, und eines Größenmaßes einer Pupille eines Auges, wie eines Pupillenradius oder -durchmessers, ausgewählt wird.

7. Computerimplementiertes Verfahren zum Bestimmen mindestens einer Augenzustandsvariablen mindestens eines Auges eines Subjekts, wobei das Auge einen Augapfel, eine eine Pupille definierende Iris und eine Hornhaut aufweist, wobei die mindestens eine Augenzustandsvariable aus mindestens einem Bild des Auges ableitbar ist, das mit einer Kamera mit bekannten Kameraeigenschaften aufgenommen wurde, wobei das Verfahren umfasst:

   - Empfangen von Bilddaten des mindestens einen Auges von einer Kamera mit bekannten Kameraeigenschaften und Definieren einer Bildebene;
   - Bestimmen einer Eigenschaft eines Pupillenbildes innerhalb der Bilddaten;
   - Bereitstellen eines 3D-Augenmodells mit mindestens einem Parameter, wobei der mindestens eine Parameter in einer vorbestimmten Beziehung von der Eigenschaft abhängt; und
   - Verwendung eines gegebenen Algorithmus zur Berechnung der mindestens einen Augenzustandsvariablen unter Verwendung der Bilddaten und des 3D-Augenmodells mit dem mindestens einen von der Eigenschaft abhängigen Parameter.

8. Verfahren nach Anspruch 7, wobei die Eigenschaft des Pupillenbildes ein Maß für die Kreisförmigkeit der Pupillenfläche oder des Pupillenumrisses ist, insbesondere ein Verhältnis zwischen der Länge der kleinen und der großen Achse einer an die Pupillenbildfläche oder den Pupillenumriss angepassten Ellipse, ein Maß für die Veränderung der Krümmung des Pupillenumrisses, ein Maß für die Dehnung oder ein Maß für die Begrenzungsbox der Pupillenfläche , und/oder wobei die vorbestimmte Beziehung zwischen dem mindestens einen Parameter des 3D-Augenmodells und der Eigenschaft des Pupillenbildes aus der Liste eines konstanten Wertes, einer linearen Beziehung, einer polynomialen Beziehung oder einer anderen nichtlinearen Beziehung, insbesondere einer über eine Regressionsanpassung abgeleiteten Beziehung, ausgewählt wird, insbesondere wobei die Beziehung in analytischer Form gespeichert und on-the-fly für gegebene Bilddaten ausgewertet oder als eine Lookup-Tabelle, LUT, gespeichert wird.

9. Verfahren nach einem der Ansprüche 7 bis 8, wobei das 3D-Augenmodell höchstens einen Parameter oder mehrere Parameter und eine vorbestimmte Beziehung zwischen einem von ihnen aufweist und die Eigenschaft für mindestens einen der Parameter verwendet wird.

10. Verfahren nach einem der Ansprüche 7 bis 9, wobei ein beliebiger Parameter des 3D-Augenmodells ausgewählt wird aus der Liste eines Abstands zwischen einem Mittelpunkt des Augapfels, insbesondere einem rotatorischen, geometrischen oder optischen Mittelpunkt, und einem Mittelpunkt einer Pupille oder Hornhaut, eines Größenmaßes eines Augapfels, einer Hornhaut oder einer Iris, wie z. B. eines Augapfelradius, eines Hornhautradius, eines Irisdurchmessers, eines Abstands zwischen Pupillenmittelpunkt und Hornhautmittelpunkt, ein Abstand Hornhaut-Mittelpunkt zu Augapfel-Mittelpunkt, ein Abstand Pupillen-Mittelpunkt zu Limbus-Mittelpunkt, ein Abstand Augenlinse zu Augapfel-Mittelpunkt, zu Hornhaut-Mittelpunkt und/oder zu Hornhaut-Scheitelpunkt, eine Brechungseigenschaft einer Augenstruktur wie ein Brechungsindex einer Hornhaut, eines Glaskörpers oder einer Augenlinse, ein Maß für die ellipsoide Form eines Augapfels oder einer Hornhaut und ein Grad des Astigmatismus , wobei die Beziehung für alle Augenzustandsvariablen die gleiche ist oder wobei eine unterschiedliche vorbestimmte Beziehung zwischen einem Parameter des 3D-Augenmodells und der Eigenschaft des Pupillenbildes für jede Augenzustandsvariable oder für Gruppen von Augenzustandsvariablen verwendet wird, und/oder wobei eine solche Beziehung gemäß dem Verfahren nach einem der Ansprüche 1 bis 6 hergestellt wurde , und/oder wobei die Augenzustandsvariable aus der Liste einer Pose eines Auges, wie einer Position eines Auges, insbesondere eines Augapfels, und/oder einer Orientierung eines Auges, insbesondere eines Blickvektors, einer optischen Achsenausrichtung oder einer Blickachsenausrichtung, einer 3D-Kreis-Mittellinie, einer 3D-Augenschnittlinie, und eines Größenmaßes einer Pupille eines Auges, wie eines Pupillenradius oder -durchmessers, ausgewählt ist.

11. Verfahren nach einem der vorhergehenden Ansprüche, wobei der gegebene Algorithmus ein Glitzern des Auges nicht berücksichtigt, um die mindestens eine Augenzustandsvariable zu berechnen, und/oder wobei der Algorithmus glitzerfrei ist, und/oder wobei der Algorithmus kein strukturiertes Licht und/oder keine spezielle Beleuchtung benötigt, um Augenzustandsvariablen abzuleiten, wobei der gegebene Algorithmus die mindestens eine Augenzustandsvariable auf eine nicht-iterative Weise berechnet, und/oder wobei der gegebene Algorithmus umfasst:

- Bestimmen einer ersten Ellipse in den Bilddaten, wobei die erste Ellipse zumindest im Wesentlichen einen Rand der Pupille des mindestens einen Auges zu einem ersten Zeitpunkt ($t_1$) darstellt;
- Verwenden der Kameraeigenschaften und der ersten Ellipse, um einen 3D-Orientierungsvektor eines ersten Kreises in 3D und eine erste Mittellinie zu bestimmen, auf der sich ein Mittelpunkt des ersten Kreises in 3D befindet, so dass erwartet wird, dass eine Projektion des ersten Kreises in einer Richtung parallel zu der ersten Mittellinie auf die Bildebene die erste Ellipse reproduziert; und
- Bestimmen einer ersten Augenschnittlinie in 3D, von der erwartet wird, dass sie einen 3D Mittelpunkt des Augapfels zu dem entsprechenden Zeitpunkt ($t_1$) schneidet, als eine Linie, die in der Richtung des Orientierungsvektors parallel zu der ersten Mittellinie um einen erwarteten Abstand zwischen dem Mittelpunkt des Augapfels und einem Mittelpunkt der Pupille verschoben ist.

12. Verfahren nach Anspruch 11, das ferner aufweist:

- Empfangen von Bilddaten eines weiteren Auges des Subjekts zu einem Zeitpunkt ($t'_1$), der im Wesentlichen dem ersten Zeitpunkt ($t_1$) entspricht, von einer Kamera mit bekannten Kameraeigenschaften, die eine Bildebene definiert, wobei das weitere Auge einen weiteren Augapfel, eine weitere Iris, die eine weitere Pupille definiert, und eine weitere Hornhaut aufweist, wobei der gegebene Algorithmus ferner umfasst:

  - Bestimmen einer weiteren Ellipse in den Bilddaten, wobei die weitere Ellipse zumindest im Wesentlichen die Grenze der weiteren Pupille des weiteren Auges zu dem entsprechenden Zeitpunkt ($t'_1$) darstellt;
  - Verwenden der Kameraeigenschaften und der weiteren Ellipse zum Bestimmen eines 3D-Orientierungsvektors eines weiteren Kreises in 3D und einer weiteren Mittellinie, auf der sich ein Mittelpunkt des weiteren Kreises in 3D befindet, so dass eine Projektion des weiteren Kreises in einer Richtung parallel zu der weiteren Mittellinie auf die Bildebene die weitere Ellipse wiedergeben soll; und
  - Bestimmen einer weiteren Augenschnittlinie in 3D, von der erwartet wird, dass sie einen 3D Mittelpunkt des weiteren Augapfels zu dem entsprechenden Zeitpunkt ($t'_1$) schneidet, als eine Linie, die in der Richtung des 3D Orientierungsvektors des weiteren Kreises parallel zu der weiteren Mittellinie um einen erwarteten Abstand zwischen dem Mittelpunkt des weiteren Augapfels und einem Mittelpunkt der weiteren Pupille verschoben ist.

13. System zur Erzeugung von Daten, die zur Bestimmung mindestens einer Augenzustandsvariablen mindestens eines Auges eines Subjekts geeignet sind, wobei das Auge einen Augapfel, eine eine Pupille definierende Iris und eine Hornhaut umfasst, wobei die mindestens eine Augenzustandsvariable aus mindestens einem Bild des Auges ableitbar ist, das mit einer Kamera mit bekannten Kameraeigenschaften aufgenommen wurde, wobei das System eine Rechen- und Steuereinheit aufweist, die konfiguriert ist, um:

- unter Verwendung der bekannten Kameraeigenschaften synthetische Bilder mehrerer Modellaugen entsprechend einem ersten 3D-Augenmodell, das die Hornhautbrechung modelliert, für eine Vielzahl gegebener Werte mindestens einer Augenzustandsvariablen zu erzeugen;
- unter Verwendung eines gegebenen Algorithmus die mindestens eine Augenzustandsvariable unter Verwendung eines oder mehrerer der synthetischen Bilder und eines weiteren 3D-Augenmodells mit mindestens einem Parameter berechnen;
- Bestimmen einer Eigenschaft eines Pupillenbildes in jedem der synthetischen Bilder;
- Bestimmen eines oder mehrerer hypothetisch optimaler Werte des mindestens einen Parameters des weiteren 3D-Augenmodells, die den Fehler zwischen dem (den) Wert(en) der mindestens einen gegebenen Augenzustandsvariablen und dem (den) Wert(en) der entsprechenden Augenzustandsvariablen, die bei Anwendung des gegebenen Algorithmus erhalten werden, minimieren; und
- Herstellen einer Beziehung zwischen dem einen oder den mehreren hypothetisch optimalen Werten des mindestens einen Parameters des weiteren 3D-Augenmodells und der Eigenschaft des Pupillenbildes und Speichern derselben in einem Speicher; und/oder wobei die Rechen- und Steuereinheit zur Durchführung des Verfahrens nach einem der Ansprüche 1 bis 6 konfiguriert ist.

14. System zur Bestimmung mindestens einer Augenzustandsvariablen mindestens eines Auges eines Subjekts, wobei das Auge einen Augapfel, eine eine Pupille definierende Iris und eine Hornhaut aufweist, wobei die mindestens eine Augenzustandsvariable aus mindestens einem Bild des Auges ableitbar ist, das mit einer Kamera mit bekannten Kameraeigenschaften aufgenommen wurde, wobei das System Folgendes umfasst:

eine Vorrichtung, die mindestens eine Kamera mit bekannten Kameraeigenschaften zum Erzeugen von Bilddaten aufweist, die mindestens ein Auge eines Subjekts einschließen, wobei die mindestens eine Kamera einen Sensor aufweist, der eine Bildebene ($_{Ip}$) definiert, wobei das mindestens eine Auge einen Augapfel, eine Iris, die eine Pupille definiert, und eine Hornhaut aufweist; und
eine Rechen- und Steuereinheit, die konfiguriert ist, um:

- Bilddaten des mindestens einen Auges von der mindestens einen Kamera zu empfangen;
- eine Eigenschaft eines Pupillenbildes innerhalb der Bilddaten zu bestimmen;
- unter Verwendung eines gegebenen Algorithmus die mindestens eine Augenzustandsvariable unter Verwendung der Bilddaten und eines 3D-Augenmodells mit mindestens einem Parameter zu berechnen, wobei der mindestens eine Parameter in einer vorbestimmten Beziehung von der Eigenschaft abhängt, wobei die Beziehung aus einem - Speicher abgerufen wird; und/oder

wobei die Rechen- und Steuereinheit zur Durchführung des Verfahrens nach einem der Ansprüche 7 bis 12 konfiguriert ist.

15. Computerprogrammprodukt und/oder computerlesbares Speichermedium, das Anweisungen aufweist, die bei Ausführung durch einen oder mehrere Prozessoren eines Systems, insbesondere des Systems nach Anspruch 13 oder Anspruch 14 , das System veranlassen, die Schritte des Verfahrens nach einem der Ansprüche 1 bis 12 auszuführen.

**Revendications**

1. Procédé, mis en œuvre par ordinateur, de génération de données pertinentes pour déterminer au moins une variable d'état d'œil d'au moins un œil d'un sujet, l'œil comprenant un globe oculaire, un iris définissant une pupille, et une cornée, l'au moins une variable d'état d'œil pouvant être dérivée d'au moins une image de l'œil prise avec une caméra ayant des caractéristiques intrinsèques de caméra connues, le procédé comprenant le fait de :

- fournir un premier modèle d'œil en 3D modélisant la réfraction cornéenne ;
- générer, à l'aide des caractéristiques intrinsèques de caméra connues, des images synthétiques de plusieurs yeux modèles selon le premier modèle d'œil en 3D, pour une pluralité de valeurs données d'au moins une variable d'état d'œil ;
- utiliser un algorithme donné pour calculer l'au moins une variable d'état d'œil à l'aide d'une ou plusieurs des images synthétiques et d'un autre modèle d'œil en 3D ayant au moins un paramètre ;
- déterminer une caractéristique d'une image de pupille dans chacune des images synthétiques ;
- déterminer une ou plusieurs valeurs hypothétiquement optimales de l'au moins un paramètre de l'autre modèle d'œil en 3D, qui minimisent l'erreur entre la ou les valeurs de l'au moins une variable d'état d'œil donnée et la ou les valeurs de la variable d'état d'œil correspondante obtenue lors de l'application de l'algorithme donné ; et
- établir une relation entre la ou les valeurs hypothétiquement optimales de l'au moins un paramètre de l'autre modèle d'œil en 3D et la caractéristique de l'image de pupille.

2. Procédé selon la revendication 1, dans lequel la caractéristique de l'image de pupille est une mesure de la circularité de la zone ou du contour de pupille, en particulier un rapport entre la longueur de l'axe mineur et la longueur de l'axe majeur d'une ellipse ajustée à la zone ou au contour d'image de pupille, une mesure d'une variation de la courbure du contour de pupille, une mesure d'allongement ou une mesure du rectangle englobant de la zone de pupille, et/ou dans lequel la relation entre les valeurs hypothétiquement optimales de l'au moins un autre paramètre de modèle d'œil en 3D et la caractéristique de l'image de pupille est choisie parmi la liste d'une valeur constante, en particulier une valeur constante inférieure ou supérieure à la valeur moyenne correspondante du premier modèle d'œil en 3D, une relation linéaire, une relation polynomiale ou une autre relation non linéaire, en particulier une relation dérivée par l'intermédiaire d'un ajustement par régression.

**3.** Procédé selon l'une quelconque des revendications précédentes, dans lequel l'autre modèle d'œil en 3D présente au plus un paramètre, ou dans lequel l'autre modèle d'œil en 3D présente plusieurs paramètres et une relation est établie pour plus d'un d'entre eux.

**4.** Procédé selon l'une quelconque des revendications précédentes, dans lequel un paramètre quelconque du premier et/ou de l'autre modèle d'œil en 3D est/sont choisi(s) dans la liste comportant une distance entre un centre d'un globe oculaire, en particulier un centre de rotation, géométrique ou optique, et un centre d'une pupille ou d'une cornée, une mesure de taille d'un globe oculaire, d'une cornée ou d'un iris, telle qu'un rayon de globe oculaire, un rayon de cornée, un diamètre d'iris, une distance entre le centre de pupille et le centre de cornée, une distance entre le centre de cornée et le centre de globe oculaire, une distance entre le centre de pupille et le centre de limbe, une distance entre le cristallin et le centre de globe oculaire, le centre de cornée et/ou le sommet de la cornée, une propriété réfractive d'une structure d'œil telle que l'indice de réfraction d'une cornée, l'humeur vitrée ou le cristallin, une mesure de forme ellipsoïdale d'un globe oculaire ou d'une cornée, et un degré d'astigmatisme.

**5.** Procédé selon l'une quelconque des revendications précédentes, dans lequel ladite relation est la même pour toutes les variables d'état d'œil, ou dans lequel une relation différente entre un paramètre de l'autre modèle d'œil en 3D et la caractéristique de l'image de pupille est établie pour chaque variable d'état d'œil ou pour des groupes de variables d'état d'œil.

**6.** Procédé selon l'une quelconque des revendications précédentes, dans lequel la variable d'état d'œil est choisie dans la liste comportant une pose d'un œil telle qu'un emplacement d'un œil, en particulier un centre de globe oculaire, et/ou une orientation d'un œil, en particulier un vecteur de regard, une orientation d'axe optique ou une orientation d'axe visuel, une ligne centrale de cercle 3D, une ligne d'intersection d'œil en 3D, et une mesure de taille d'une pupille d'un œil, telle qu'un rayon ou un diamètre de pupille.

**7.** Procédé, mis en œuvre par ordinateur, de détermination d'au moins une variable d'état d'œil d'au moins un œil d'un sujet, l'œil comprenant un globe oculaire, un iris définissant une pupille, et une cornée, l'au moins une variable d'état d'œil pouvant être dérivée d'au moins une image de l'œil prise avec une caméra ayant des caractéristiques intrinsèques de caméra connues, le procédé comprenant le fait de :

- recevoir des données d'image de l'au moins un œil provenant d'une caméra ayant des caractéristiques intrinsèques de caméra connues et définissant un plan d'image;
- déterminer une caractéristique d'une image de pupille dans les données d'image ;
- fournir un modèle d'œil en 3D ayant au moins un paramètre, l'au moins un paramètre dépendant d'une relation prédéterminée avec la caractéristique ; et
- utiliser un algorithme donné pour calculer l'au moins une variable d'état d'œil à l'aide des données d'image et du modèle d'œil en 3D comprenant l'au moins un paramètre dépendant de la caractéristique.

**8.** Procédé selon la revendication 7, dans lequel la caractéristique de l'image de pupille est une mesure de la circularité de la zone ou du contour de pupille, en particulier un rapport entre la longueur de l'axe mineur et la longueur de l'axe majeur d'une ellipse ajustée à la zone ou au contour d'image de pupille, une mesure d'une variation de la courbure du contour de pupille, une mesure d'allongement ou une mesure du rectangle englobant de la zone de pupille, et/ou dans lequel la relation prédéterminée entre l'au moins un paramètre du modèle d'œil en 3D et la caractéristique de l'image de pupille est choisie dans la liste comportant une valeur constante, une relation linéaire, une relation polynomiale, ou une autre relation non linéaire, en particulier une relation dérivée par l'intermédiaire d'un ajustement par régression, en particulier dans lequel la relation est stockée sous une forme analytique et évaluée à la volée pour des données d'image données, ou est stockée sous la forme d'une table de consultation, LUT.

**9.** Procédé selon l'une quelconque des revendications 7 à 8, dans lequel le modèle d'œil en 3D présente au plus un paramètre ou présente plusieurs paramètres et une relation prédéterminée entre l'un quelconque d'entre eux et la caractéristique est utilisée pour au moins l'un des paramètres.

**10.** Procédé selon l'une quelconque des revendications 7 à 9, dans lequel un paramètre quelconque du modèle d'œil en 3D est choisi dans la liste comportant une distance entre un centre d'un globe oculaire, en particulier un centre de rotation, géométrique ou optique, et un centre d'une pupille ou d'une cornée, une mesure de taille d'un globe oculaire, d'une cornée ou d'un iris, tel qu'un rayon de globe oculaire, un rayon de cornée, un diamètre d'iris, une distance entre le centre de pupille et le centre de cornée, une distance entre le centre de cornée et le centre de globe oculaire, une distance entre le centre de pupille et le centre de limbe, une distance entre le cristallin et le centre de globe oculaire, le centre de cornée et/ou le sommet de la cornée, une propriété réfractive d'une structure d'œil telle que l'indice de réfraction d'une cornée, l'humeur vitrée ou le cristallin, une mesure de forme ellipsoïdale d'un globe oculaire ou d'une cornée, et un degré d'astigmatisme, dans lequel ladite relation est la même pour toutes les variables d'état d'œil, ou dans lequel une relation prédéterminée différente entre un paramètre du modèle d'œil en 3D et la caractéristique de l'image de pupille est utilisée pour chaque variable d'état d'œil ou pour des groupes de variables d'état d'œil, et/ou dans lequel une telle relation a été établie conformément au procédé selon l'une quelconque des revendications 1 à 6, et/ou dans lequel la variable d'état d'œil est choisie dans la liste comportant une pose d'un œil telle qu'un emplacement d'un œil, en particulier un centre de globe oculaire, et/ou une orientation d'un œil, en particulier un vecteur de regard, une orientation d'axe optique ou une orientation d'axe visuel, une ligne centrale de cercle 3D, une ligne d'intersection d'œil en 3D, et une mesure de taille d'une pupille d'un œil, telle qu'un rayon ou un diamètre de pupille.

11. Procédé selon l'une quelconque des revendications précédentes, dans lequel l'algorithme donné ne tient pas compte d'un reflet provenant de l'œil pour calculer l'au moins une variable d'état d'œil, et/ou dans lequel l'algorithme est exempt de reflets, et/ou dans lequel l'algorithme ne nécessite pas de lumière structurée et/ou d'éclairage spécial pour dériver des variables d'état d'œil, dans lequel l'algorithme donné calcule l'au moins une variable d'état d'œil de manière non itérative, et/ou dans lequel l'algorithme donné comprend le fait de :

- déterminer une première ellipse dans les données d'image, la première ellipse représentant au moins sensiblement une bordure de la pupille de l'au moins un œil à un premier instant ($t_1$) ;
- utiliser les caractéristiques intrinsèques de caméra et la première ellipse pour déterminer un vecteur d'orientation 3D d'un premier cercle en 3D et une première ligne centrale sur laquelle un centre du premier cercle est localisé en 3D, de sorte qu'une projection du premier cercle, dans une direction parallèle à la première ligne centrale, sur le plan d'image est susceptible de reproduire la première ellipse ; et
- déterminer une première ligne d'intersection d'œil en 3D susceptible de croiser un centre 3D du globe oculaire à l'instant correspondant ($t_1$) sous la forme d'une ligne qui, dans la direction du vecteur d'orientation, est décalée parallèlement à la première ligne centrale d'une distance attendue entre le centre du globe oculaire et un centre de la pupille.

12. Procédé selon la revendication 11, comprenant en outre le fait de :

- recevoir des données d'image d'un autre œil du sujet à un instant ($t'_1$) correspondant sensiblement au premier instant ($t_1$), en provenance d'une caméra ayant des caractéristiques intrinsèques de caméra connues et définissant un plan d'image, l'autre œil comprenant un autre globe oculaire, un autre iris définissant une autre pupille et une autre cornée, l'algorithme donné comprenant en outre le fait de :

- déterminer une autre ellipse dans les données d'image, l'autre ellipse représentant au moins sensiblement la bordure de l'autre pupille de l'autre œil à l'instant correspondant ($t'_1$) ;
- utiliser les caractéristiques intrinsèques de caméra et l'autre ellipse pour déterminer un vecteur d'orientation 3D d'un autre cercle en 3D et une autre ligne centrale sur laquelle un centre de l'autre cercle est situé en 3D, de sorte qu'une projection de l'autre cercle, dans une direction parallèle à l'autre ligne centrale, sur le plan d'image, est susceptible de reproduire l'autre ellipse ; et
- déterminer une autre ligne d'intersection d'œil en 3D susceptible de croiser un centre 3D de l'autre globe oculaire à l'instant correspondant ($t'_1$) sous la forme d'une ligne qui, dans la direction du vecteur d'orientation 3D de l'autre cercle, est décalée parallèlement à l'autre ligne centrale d'une distance attendue entre le centre de l'autre globe oculaire et un centre de l'autre pupille.

13. Système destiné à générer des données pertinentes pour déterminer au moins une variable d'état d'œil d'au moins un œil d'un sujet, l'œil comprenant un globe oculaire, un iris définissant une pupille, et une cornée, l'au moins une variable d'état d'œil pouvant être dérivée d'au moins une image de l'œil prise avec une caméra ayant des caractéristiques intrinsèques de caméra connues, le système comprenant une unité de calcul et de commande configurée pour :

- générer, à l'aide des caractéristiques intrinsèques de caméra connues, des images synthétiques de plusieurs yeux modèles selon un premier modèle d'œil en 3D modélisant la réfraction cornéenne, pour une pluralité de valeurs données d'au moins une variable d'état d'œil ;
- calculer, en utilisant un algorithme donné, l'au moins une variable d'état d'œil à l'aide d'une ou plusieurs des images synthétiques et d'un autre modèle d'œil en 3D ayant au moins un paramètre ;
- déterminer une caractéristique d'une image de pupille dans chacune des images synthétiques ;

- déterminer une ou plusieurs valeurs hypothétiquement optimales de l'au moins un paramètre de l'autre modèle d'œil en 3D, qui minimisent l'erreur entre la ou les valeurs de l'au moins une variable d'état d'œil donnée et la ou les valeurs de la variable d'état d'œil correspondante obtenue lors de l'application de l'algorithme donné ; et
- établir une relation entre la ou les valeurs hypothétiquement optimales de l'au moins un paramètre de l'autre modèle d'œil en 3D et la caractéristique de l'image de pupille, et la stocker dans une mémoire ; et/ou

dans lequel l'unité de calcul et de commande est configurée pour mettre en œuvre le procédé selon l'une quelconque des revendications 1 à 6.

**14.** Système destiné à déterminer au moins une variable d'état d'œil d'au moins un œil d'un sujet, l'œil comprenant un globe oculaire, un iris définissant une pupille, et une cornée, l'au moins une variable d'état d'œil pouvant être dérivée d'au moins une image de l'œil prise avec une caméra ayant des caractéristiques intrinsèques de caméra connues, le système comprenant :

un dispositif comprenant au moins une caméra ayant des caractéristiques intrinsèques de caméra connues pour produire des données d'image comprenant au moins un œil d'un sujet, l'au moins une caméra comprenant un capteur définissant un plan d'image ($I_p$), l'au moins un œil comprenant un globe oculaire, un iris définissant une pupille, et une cornée ; et
une unité de calcul et de commande configurée pour :

- recevoir des données d'image de l'au moins un œil en provenance de l'au moins une caméra ;
- déterminer une caractéristique d'une image de pupille dans les données d'image ;
- calculer, en utilisant un algorithme donné, l'au moins une variable d'état d'œil à l'aide des données d'image et d'un modèle d'œil en 3D ayant au moins un paramètre, l'au moins un paramètre dépendant d'une relation prédéterminée avec la caractéristique, la relation étant récupérée à partir d'une mémoire ; et/ou

dans lequel l'unité de calcul et de commande est configurée pour mettre en œuvre le procédé selon l'une quelconque des revendications 7 à 12.

**15.** Produit-programme informatique et/ou support de stockage lisible par ordinateur comprenant des instructions qui, lorsqu'elles sont exécutées par un ou plusieurs processeurs d'un système, en particulier le système selon la revendication 13 ou 14, amènent le système à mettre en œuvre les étapes du procédé selon l'une quelconque des revendications 1 à 12.

FIG 1A

FIG 1B

FIG 1C

FIG 2A

FIG 2B

FIG 2C

FIG 3

FIG 4A

FIG 4B

FIG 4C

FIG 5A

FIG 5B

FIG 5C

FIG 6A

FIG 6B

1000

2000

```
┌──────────────┐                           ┌──────────────┐
│     1100     │                           │     2100     │
└──────────────┘                           └──────────────┘
   │ I_k                                      │ n_ref   {X_gt}
   ▼                                          ▼
┌──────────────┐                           ┌──────────────┐
│     1200     │                           │     2200     │
└──────────────┘                           └──────────────┘
   │ c_k                                      │ SI_i
   ▼                      ┌──────────────┐    ▼
┌──────────────┐          │     2410     │ ┌──────────────┐
│     1300     │◄╌╌╌╌╌╌   └──────────────┘ │     2300     │
└──────────────┘                │          └──────────────┘
{R'_opt(c_k)}  │ I_k            │ R          │ c_i   SI_i
               ▼                └────────►   ▼
┌──────────────┐                           ┌──────────────┐
│     1400     │                           │     2420     │
└──────────────┘                           └──────────────┘
   │                                       {X_gt}    {X_ex}
   ▼                                          ▼
 {X_k}                                     ┌──────────────┐
                                           │     2500     │
                                           └──────────────┘
                                             c_i    {R'_opt,i}
                                                ▼
                                           ┌──────────────┐
                                           │     2600     │
                                           └──────────────┘
                                                │
                                                ▼
                                             {R'_opt(c)}
```

FIG 7A                                   FIG 7B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020244752 A

- WO 2020244971 A

**Non-patent literature cited in the description**

- **KAI DIERKES et al.** A fast approach to refraction-aware eye-model fitting and gaze prediction. *ETRA '19: Proceedings of the 11th ACM Symposium on Eye Tracking Research & Applications Article*, vol. 23, 1-9
- **SWIRSKI L. et al.** A fully-automatic, temporal approach to single camera, glint-free 3D eye model fitting. *Proc. PETMEI Lund/Sweden*, 13 August 2013
- **NAVARRO R. et al.** Accommodation-dependent model of the human eye with aspherics. *J. Opt. Soc. Am. A*, 1985, vol. 2 (8), 1273-1281

- **SAFAEE-RAD R. et al.** Three-dimensional location estimation of circular features for machine vision. *IEEE Transactions on Robotics and Automation*, 1992, vol. 8 (5), 624-640
- **DIERKES K. et al.** A fast approach to refraction-aware eye-model fitting and gaze prediction. *Proc. ETRA Denver/USA*, 25 June 2019
- **FEDTKE C. et al.** The entrance pupil of the human eye: a three-dimensional model as a function of viewing angle. *Optics Express*, 2010, vol. 18 (21), 22364-22376